# EUROPEAN PATENT APPLICATION

(11) **EP 2 147 673 A1**
(43) Date of publication of application: **27.01.2010**
(21) Application number: 09012181.5
(22) Date of filing: 29.11.2007
(51) Int. Cl.: A61K 31/566, A61K 31/568, A61K 31/5685, A61P 1/00, A61P 3/10, A61P 9/00, A61P 11/06, A61P 17/02, A61P 25/02, A61P 25/28, A61P 29/00, A61P 35/00

(54) **Modulation of prostaglandin/cyclooxygenase metabolic pathways**

(30) Priority: 30.11.2006 GB 0623971; 30.11.2006 US 867873 P
(62) Divisional of application: 07870407.9
(71) Applicant: Hunter-Fleming Limited, Bristol BS1 6EA (GB)
(72) Inventor: Wülfert, Ernst, 1180 Brussels (BE)
(74) Representative: Fairbairn, Angus Chisholm

(57) **Abstract**

A variety of diseases and disorders associated with the metabolic pathways involved in the activities of cyclooxygenase and the synthesis of prostaglandins, for example type 2 diabetes mellitus and its sequelae, ischemic vascular diseases, pain associated with inflammation, inflammatory skin conditions, spinal cord injury, peripheral neuropathy, multiple sclerosis, inflammatory bowel disease and rheumatoid arthritis, as well as various types of cancer may be treated or prevented by the use of compounds as agents which selectively enhance production of 15-deoxy-prostaglandin J2. The compounds are 7-hydroxy steroids of formula (IV) or a salt or ester thereof: 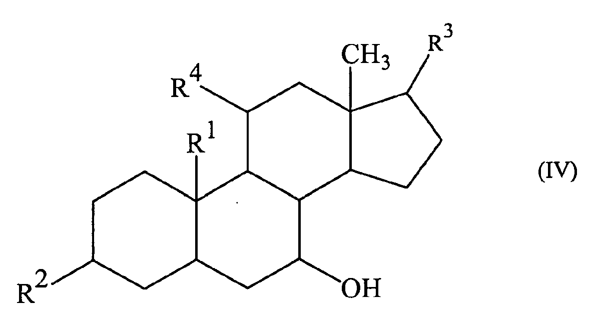 wherein R¹ represents hydrogen or methyl and R², R³ and R⁴ each independently represents an oxo, hydroxy, mercapto, hydrogen, halogen, alkoxy, aryloxy or acyl group.

## Description

The present invention relates to a series of compounds which we have discovered modulate the metabolic pathways involved in the activities of cyclooxygenase and prostaglandin synthases and the synthesis of prostaglandins , and which, as a result, may be used in the treatment and prophylaxis of a variety of diseases and disorders, some of which have proved very refractory to previous treatment regimes.

Prostaglandins (PGs), also referred to as prostanoids, are a widely distributed group of oxygenated lipids that modulate cellular functions in both a physiological and pathological context. The biosynthesis of PGs is initiated through the release of arachidonic acid (AA), a major fatty acid, from cell membranes, a reaction catalyzed by phospholipase A2 (PLA2). Released AA is converted to an unstable oxygenated intermediate (PGH2) by cyclooxygenase (COX). Once formed, the PGH2 intermediate may be converted into various prostaglandins such as prostaglandin E2 (PGE2), prostacyclin (PGI2), prostacyclin F2α (PGF2α) or prostaglandin D2 (PGD2), by a range of specific enzymes called prostaglandin synthases (e.g. PGE synthase or PGE-S, PGD synthase or PGD-S etc.).

COX is present in three isoforms: COX-1, COX-2 and the COX-1 splice variant COX-3. COX-1 is constitutively expressed in most tissues, whereas COX-2 is generally induced in response to pro-inflammatory cytokines and stress. These findings led to the straightforward view that COX-2 is responsible for the adverse pro-inflammatory effects of prostanoids. Therefore, inhibition of COX-2 has been considered a prime target in developing drugs for the treatment of inflammatory diseases. However, investigators have shown that COX-2 also plays an important role in basal organ and tissue homeostasis.

Recent clinical trials have revealed that long-term treatment with COX-2 inhibitors increases the incidence of stroke and myocardial infarction, complications attributed to blockade of the vasoprotective effects of COX-2-derived PGI2. However, blockade of COX enzymes also reduces the production of PGD2. In peripheral tissues, PGD2 promotes vasodilatation and inhibits platelet aggregation. In the brain, it is the most abundant prostaglandin, and a recent study shows that PGD2 mediates neuroprotective effects in hippocampal neurones. Blockade of COX-2-derived PGD2 may therefore contribute to the increase in the incidence of stroke and myocardial infarction observed in clinical trials with COX-2 inhibitors.

PGD2 is very short lived, and undergoes dehydration *in vivo* and *in vitro* to yield biologically active prostaglandins of the J2 series, including prostaglandin 15-deoxy-prostaglandin J2 (15d-PGJ2). This prostaglandin is a natural, chemically stable anti-inflammatory derivative of PGD2. 15d-PGJ₂ is a high-affinity ligand for the peroxisome proliferator-activated receptor (PPAR) subtype PPARγ, which is a ligand-dependent nuclear transcription factor that has been implicated in a broad range of cellular functions, including anti-inflammatory actions. 15d-PGJ2 represses several inflammatory genes such as nitric oxide synthase, prostaglandin E synthase (PGES) and tumour necrosis factor-α (TNFα) genes through PPAR-γ-dependent as well as PPARγ-independent mechanisms. In rat chondrocytes, 15d-PGJ2 decreased almost completely cytokine stimulated PGE2 synthesis and PGES expression, indicating that 15d-PGJ2 is an anti-inflammatory messenger that turns off the production of the pro-inflammatory prostaglandin PGE2 in these cells. Regardless of the mechanism, 15d-PGJ2 is present *in vivo* during the resolution phase of inflammation, again suggesting that it may function as a feedback regulator of the inflammatory response. Administration of 15d-PGJ2 has also been shown to reduce the development of experimental colitis in the rat, a model of inflammatory bowel disease. Agents and conditions that tip the balance of prostaglandin production in favour of PGD2 and 15d-PGJ2 would therefore be expected to have anti-inflammatory effects.

In the central nervous system, the anti-inflammatory actions of 15d-PGJ2 could be beneficial in neurodegenerative disorders such as Alzheimer's disease, Parkinson's disease and multiple sclerosis, but also in stroke, spinal cord injury and brain trauma where inflammation contributes to brain damage and cell death. In all of these conditions, brain damage is associated with excessive microglial activation. In the central nervous system, microglia, the resident innate immune cells, play a major role in the inflammatory process. Uncontrolled activation of microglia may be directly toxic to brain cells by releasing various substances such as inflammatory cytokines (IL-1β, TNF-α, IL-6), NO, PGE2, and superoxide. Several studies show that 15d-PGJ2 can repress the production of inflammatory cytokines and NO by activated microglial cells and astrocytes, suggesting that the prostaglandins could play an important role in preventing brain damage associated with excessive glial cell activation. The finding that administration of 15d-PGJ2 before and at the onset of experimental autoimmune encephalitis (EAE), an animal model for multiple sclerosis, significantly reduced the severity of EAE further suggests that 15d-PGJ2 may be effective in preventing brain damage in inflammatory neurodegenerative diseases.

Recent experimental studies show that 15d-PGJ2 reduces brain tissue inflammation and behavioural dysfunction and neuronal loss after intra-cerebral haemorrhage in rats, and protects the brain from ischaemia-reperfusion injury in an experimental model of stroke. Intra-ventricular infusion of 15d-PGJ2 reduced brain infarct volume, inhibited brain and neuronal apoptosis, suppressed NF-κB activation, and upregulated haeme oxygenase-1 (HO-1), a powerful endogenous anti-oxidant, in a PPARγ-dependent manner. These results, which suggest a neuroprotective effect of 15d-PGJ2 in ischaemic stroke, are further corroborated by a recent study showing that patients with acute ischaemic stroke have higher plasma levels of 15d-PGJ2 than normal subjects and that increased plasma 15d-PGJ2 is associated with better early and late neurological outcome. Increased plasma 15d-PGJ2 was also associated with reduced infarct volume, and this effect was independent of the effect of other important prognostic variables. Agents and conditions that shift the balance of prostaglandin production in favour of 15d-PGJ2 would therefore be expected to have beneficial effects both in chronic inflammatory neurodegenerative disorders such as Alzheimer's Disease (AD), Parkinson's disease (PD) and multiple sclerosis, and in acute conditions such as stroke, spinal cord injury and traumatic brain injury.

AD is a progressive and fatal neurodegenerative disorder characterized by the deposition of extracellular Aβ (amyloid-β) plaques and the formation of intracellular tangles in the brain. Aβ plaques are mainly composed of amyloid-β and Aβ peptides. Aβ accumulation causes an inflammatory response that has been proposed to contribute to the pathogenesis of AD and to increase neuronal damage. Increased brain levels of soluble Aβ-peptides are also thought to cause neuronal dysfunction and cognitive impairment long before plaque deposition takes place. The formation of Aβ peptides in the brain is therefore a prime trigger of the pathogenesis of AD.

The generation of Aβ is initiated by a protease that cleaves a larger precursor protein, the β-amyloid precursor protein (βAPP), at the N terminus side of the Aβ peptide. This protease, also called β-secretase or β-site APP-cleaving enzyme (BACE1), is a trans-membrane aspartyl protease. BACE1 protein levels and the β-secretase product (β-C-terminal fragment) are increased in brain of sporadic AD patients. Reducing Aβ production in the AD brain is therefore a main therapeutic objective.

It has been reported that BACE1 mRNA and protein levels are increased by pro-inflammatory mediators and down regulated by drugs that are agonists for peroxisome proliferator-activated receptor-γ (PPARγ). More recently, it has also been shown that depletion of PPARγ potentiates β-secretase mRNA levels by increasing BACK1 gene promoter activity, whereas over-expression of PPARγ, as well as PPARγ activators, specifically modulate BACE1 transcription by repressing BACE1 gene promoter activity, suggesting that PPARγ could be a repressor of BACE1. Furthermore, treatment of transgenic hAPP-mice with PPARγ agonists reduced both BACE1 mRNA and intracellular β-amyloid levels in cortical neurones.

Over-expression of PPARγ has also been shown to reduce Aβ secretion in cultured cells via activation of ubiquitination and proteasome-mediated degradation of the precursor protein βAPP, and activation of PPARγ was reported to directly affect the stability of Aβ externally added to cells in culture. This decrease in stability suggests that PPARγ activation may induce a rapid cellular clearance mechanism for amyloid peptide.

Altogether, these data support a major role for PPARγ in the modulation of amyloid-β generation, and suggest a protective mechanism through which activation of PPARγ decreases the production of amyloid-β peptides in the brain.

15d-PGJ_{2,} a high-affinity ligand for the peroxisome proliferator-activated receptor (PPAR) subtype PPARγ, represses several inflammatory genes through PPARγ-dependent as well as PPARγ-independent mechanisms. Conditions that increase the endogenous production of 15d-PGJ2 would therefore be expected both to suppress brain inflammatory processes, and to reduce amyloid peptide formation in the AD brain. Consequently, agents and conditions that increase the endogenous production of 15d-PGJ2 would be expected to have beneficial effects in the treatment of Alzheimer's disease.

Prostaglandins of the J2 series are powerful inducers of nerve growth factor (NGF) and brain derived nerve growth factor (BDNF) production, and promote neurite outgrowth by NGF in cell culture. The neurite promoting ability of 15d-PGJ2 does not occur through PPARγ because synthetic PEAR-γ agonist and antagonist did not change the neurite-promoting effect of 15-deoxy-PGJ_{2.} In animal studies, intra-cerebroventricular administration of NGF has been shown to rescue cholinergic neurons, stimulate axonal growth and improve cholinergic function. Similarly, intra-cerebroventricular injection of NGF attenuated neuronal death in the hippocampi of gerbils subjected to brain ischemia. BDNF has been shown to promote the survival and growth of developing neurons *in vitro,* and to improve motor neuronal functions in animal models. In animals subjected to transient forebrain ischemia, BDNF attenuated ischemic neuronal injury. Unfortunately, due to poor penetrations of these neurotrophins across the blood brain barrier, clinical trials have failed to show significant effects. However, conditions or treatments that increase endogenous levels of 15d-PGJ2 would be expected both to facilitate local production of these growth factors and to promote neuronal growth and hence facilitate neuronal repair in the spinal cord and in the brain.

Increased levels of inflammatory markers are associated with ischemic vascular disease, and inflammation is increasingly considered to be involved in the pathogenesis of acute coronary syndromes. Inflammation has a relevant role in the initiation and progression of atherosclerosis, but it can also play a primary role in thrombosis development by activating the coagulation process. Conditions and agents that reduce vascular inflammation could therefore be beneficial in cardiovascular disorders in which inflammation contributes to cell death or damage, such as coronary heart disease.

Prostaglandin D synthase (PGDS) mRNA is expressed in the heart. Thus, locally produced PGD₂ may result in 15d-PGJ₂ in myocytes or surrounding cells. PPARγ is also present and functional in cardiac myocytes. A recent study showed that 15d-PGJ₂, the natural metabolite of PGD₂, exerts anti-inflammatory effects in cardiac myocytes by modulating IL-1β-stimulated COX-2, PGE-S, and iNOS in a PPAR-dependent manner. 15d-PGJ₂ blocked IL-1ß stimulation of PGE₂ production but did not modify IL-1β stimulation of PGI₂ or PGF_{2α}, indicating that PPARγ ligand effects are specific for PGE-S. Blockade of IL-1β-induced PGE-S by 15d-PGJ₂ would be expected to decrease the production of the pro-inflammatory prostaglandin PGE2 in cardiac tissue. 15d-PGJ₂ also been shown to up-regulate the expression of haeme-oxygenase-1 (HO-1) in cardiac myocytes and to reduce myocardial infarct size in a rat model of regional ischaemia-reperfusion-induced myocardial infarction. Among the different PPARγ ligands studied, 15d-PGJ2 caused by far the most pronounced reduction in infarct size. Whereas this effect is mediated through PPARγ, increased expression of the antioxidant and cytoprotective protein HO-1 was PPARγ-independent. Altogether, these results suggest that conditions and agents that increase endogenous levels of 15d-PGJ2 could reduce vascular inflammation and therefore be beneficial in cardiovascular disorders.

Increasing evidence identifies adipose tissue as a major source of circulating inflammatory factors, particularly in the presence of obesity. Fat produces pro-inflammatory adipocytokines, which include TNF-α leptin, PAI-1, IL-6, and angiotensinogen. TNF-α is a major activator of NFκB. TNF-α also inhibits insulin signalling, thereby causing insulin resistance. PAI-1 levels predict CAD and diabetes, and they are a major contributor to the pro-thrombotic state in obesity. IL-6 stimulates liver production of C-reactive protein (CRP) and contributes to the elevated highly sensitive (hs)CRP levels in serum of obese subjects. HsCRP predicts myocardial infarction, stroke, peripheral arterial disease, and sudden death. Angiotensinogen is the precursor of Ang II, which is well known to activate multiple mechanisms of vascular injury. In general, all of these adipokines are elevated in insulin-resistant subjects with increased visceral adiposity creating a proinflammatory milieu. Type 2 diabetes mellitus (T2D) and obesity is therefore an inflammatory condition.

Adipose tissue expresses the highest levels of PPARγ compared with other tissues. PPARγ ligands promote fat cell differentiation and uptake of free fatty acids into adipose tissue. They also have an important effect to attenuate the pro-inflammatory milieu by decreasing expression of TNF-α PAI-1, and IL-6 and increasing adiponectin expression in fat. Thus, PPARγ activation suppresses inflammation directly in vascular cells and indirectly through regulation of gene expression in adipose tissue.

T2D and the metabolic syndrome are characterized by resistance to the action of insulin in peripheral tissues, including skeletal muscle, liver and adipose tissue. Activation of PPARγ by certain of the synthetic ligands, such as thiazolidinediones, improves insulin sensitivity and lowers circulating levels of glucose, triglycerides and free fatty acids without stimulating insulin secretion in rodent models of T2D. PPARγ agonists also alleviate peripheral insulin resistance in humans, and have been effectively used in the treatment of T2D patients.

15-deoxy-prostaglandin J2 (15d-PGJ2) is a natural, chemically stable anti-inflammatory prostaglandin that appears to be the putative endogenous, high-affinity ligand for the peroxisome proliferator-activated receptor subtype PPARγ. Agents and conditions that increase the endogenous production of 15d-PGJ2 would therefore be expected both to suppress vascular inflammation and improve insulin sensitivity in Type 2 diabetes.

Pro-inflammatory cytokines, such as tumour necrosis factor (TNF)-alpha are over-expressed in psoriasis and atopic dermatitis. TNF-α plays an important role both in the initiation and persistence of inflammation, and recent experimental data show that the development of lesions in an experimental model of psoriasis is mediated by TNF-α These findings, which suggest a role for TNF-α in the pathogenesis of psoriasis, are supported by results from recent clinical trials, showing that administration of a monoclonal antibody (mAb) against TNF-α (infliximab) or a soluble TNF receptor fusion protein (etanercept) resulted in disease improvement in psoriasis patients.

15d-PGJ₂, a chemically stable metabolite of prostaglandin PGD2, is a high-affinity ligand for the peroxisome proliferator-activated receptor γ (PPARγ).

15d-PGJ₂ represses several pro-inflammatory genes in activated macrophages, in microglial cells and in human astrocytes, including the inducible NO synthase (iNOS) and tumour necrosis factor α (TNF-α) genes, and this repression is at least partly dependent on PPARγ expression. Moreover, synthetic PPARγ ligands, such as the insulin-sensitizing thiazolidinediones, have been shown to improve psoriasis in human subjects. Altogether, these findings suggest that agents and conditions that increase the endogenous production of 15d-PGJ₂ could be effective in the treatment of psoriasis.

Recent evidence indicates that certain synthetic PPARγ agonists exhibit moderate anti-proliferative activities against many epithelial-derived human cancer cell lines. Moreover, recent data indicate that normal prostate epithelial cells and T lymphocytes are more resistant to apoptotic induction by these PPARγ ligands. In the light of this cancer-specific effect, the potential use of PPARγ agonists as chemo-preventive agents has received much attention.

15d-PGJ_{2,} a natural ligand for PPARγ, has also been shown to possess anti-tumour activity. For example, 15d-PGJ₂ significantly inhibits cell growth and induces apoptosis in several types of cancer cells, including colorectal, gastric, breast, and hepatic cancer cells. Mechanistic studies suggest that these growth inhibitory effects are mediated through PPARγ-independent mechanisms. However, regardless of the mechanism involved, agents or conditions that increase the endogenous levels of 15d-PGJ₂ would be expected to inhibit tumour growth and progression.

Increased levels of prostaglandin E₂ (PGE₂) have been detected in a variety of malignancies. Several lines of evidence, beyond the finding of elevated levels of PGE₂ in tumours, suggest that PGE₂ plays a role in the development and progression of cancer. For example, PGE₂ can stimulate cell proliferation and motility while inhibiting apoptosis and immune surveillance. Importantly, PGE₂ can also induce angiogenesis, at least in part, by enhancing the production of pro-angiogenic factors including vascular endothelial growth factor. Consistent with these findings, higher levels of PGE₂ in cancer tissue specimens have been shown to correlate significantly with the occurrence of metastatic disease and increased tumour vascularisation.

Recent work in experimental animals also suggests that PGE₂ can promote carcinogenesis. Genetic disruption of the PGE₂ receptor EP₂ was found to decrease the number and size of experimental tumours, and other studies showed that treatment with anti-PGE₂ monoclonal antibody inhibited the growth of transplantable tumours. Given this background, one might expect that agents and conditions that inhibit the enzymatic pathways that lead to increased amounts of PGE₂ in cancer also inhibit the progression of tumour growth.

The synthesis of PGE₂ from arachidonic acid requires two enzymes that act in sequence, cyclooxygenase (COX) and prostaglandin E synthase (PGES). Increased expression of PGES has been detected in several human malignancies, raising the possibility that aberrant PGES expression drives the increased production of PGE₂ that contributes to cell proliferation and tumour growth. 15d-PGJ2 has been reported to almost completely inhibit cytokine-induced PGE2 synthesis and membrane PGE-synthase expression. Consequently, agents and conditions that increase the endogenous levels of 15d-PGJ₂ would be expected to reduce PGE₂ synthesis and hence cell proliferation and tumour growth, and therefore have beneficial effects in the treatment of cancer.

We have previously shown that 7β-hydroxy-EPIAndrosterone (7β-OH-EPIA), an endogenous 7-hydroxysteroid, has both neuroprotective and cardioprotective effects (WO 02/00224, WO 02/00225 and WO 03/015791). The steroid protects against neuronal cell death *in vitro* (organotypic hippocampal slice cultures (OTHSC) and PC12 cells) and against brain damage in numerous *in vivo* experimental models, and is effective in protecting against regional ischaemia-induced myocardial infarction in perfused rat hearts. Altogether, these findings suggest that 7β-OH-EPIA could have beneficial effects in the prevention and treatment of neurodegenerative conditions such as stroke, spinal cord injury, traumatic brain injury and AD, and in cardiovascular conditions such as myocardial infarction (MI).

More recently, we have shown that incubation of PC12 cells with indomethacin, a cyclooxygenase (COX) inhibitor, completely blunted 7β-OH-EPIA-induced protection against ischaemia. These results suggest that COX activity is required for the neuroprotective effects of 7β-OH-EPIA.

We now show that incubation of human monocytic blood cells (hMBC) with nanomolar concentrations of 7β-OH-EPIA causes an almost 10-fold increase in prostaglandin 15deoxy-Δ12,14-J2 (15d-PGJ2) production. This effect of the 7-hydroxysteroid appears to be specific for 15d-PGJ2, since the steroid did not significantly alter the production of prostaglandin E2 (PGE2) in these cells. In contrast, incubation of hMBC with the pro-inflammatory cytokine tumour necrosis factor-α (TNFα) increased the production of both prostaglandins approx. 3-fold. Moreover, co-incubation with TNF-α and 7β-OH-EPIA caused an increase in 15d-PGJ2 similar to the increase observed with 7β-OH-EPIA alone, whereas addition of nanomolar concentrations of 7β-OH-EPIA completely abolished the increases in PGE2 by TNFα

15d-PGJ2 has been reported to almost completely inhibit cytokine-induced PGE2 synthesis and membrane PGE-synthase (mPGES) expression in rat chondrocytes, indicating that 15d-PGJ2 is an anti-inflammatory messenger that turns off the production of the pro-inflammatory prostaglandin PGE2 in these cells. Our findings may therefore indicate that inhibition of TNFα-induced production of the pro-inflammatory prostaglandin PGE2 by 7β-OH-EPIA is mediated through increased release of 15d-PGJ2.

15d-PGJ₂ is a high-affinity ligand for the peroxisome proliferator-activated receptor (PPAR) subtype PPARγ, a ligand-dependent nuclear transcription factor that has been implicated in a broad range of cellular functions, including anti-inflammatory, neuroprotective, cardioprotective, metabolic and anti-tumour actions. We have now discovered that agents such as 7β-OH-EPIA that can selectively facilitate 15d-PGJ₂ production and so could be beneficial in inflammation and skin disorders such as inflammatory bowel disease and psoriasis, and in neurological, cardiovascular and metabolic disorders such as stroke, spinal cord injury, traumatic brain injury, AD, PD, coronary heart disease and Type 2 diabetes in which inflammation contributes to dysfunction and cell death, and in various types of cancer in which increased production of PGE2 contributes to cell proliferation and tumour growth.

Thus, in one aspect, the present invention consists in the use of a compound of formula (IV): 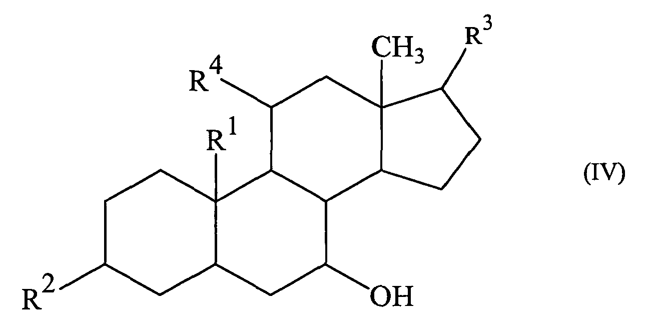 wherein:
R¹ represents a hydrogen atom or a methyl group; and
R², R³ and R⁴ are the same as or different from each other and each represents an oxo group, a hydroxy group, a mercapto group, a hydrogen atom, a halogen atom, an alkoxy group, an aryloxy group or an acyl group; or a pharmaceutically acceptable salt or ester thereof,

as an agent which enhances production of 15-deoxy-prostaglandin J2 for the manufacture of a medicament for the treatment or prophylaxis of conditions mediated by enhanced levels of prostaglandin E2 or other metabolites of cyclooxygenase and prostaglandin synthase activity or for the treatment or prophylaxis of conditions made worse due to a reduced level or reduced availability of 15-deoxy-prostaglandin J2.

In a further aspect, the present invention consists in the use of a compound of formula (IV) as defined above as an agent which facilitates production of 15-deoxy-prostaglandin J2 and selectively inhibits production of prostaglandin E2 in the presence of an inflammation causing agent for the manufacture of a medicament for the treatment or prophylaxis of conditions mediated by enhanced levels of prostaglandin E2 or other metabolites of cyclooxygenase-2 or for the treatment or prophylaxis of conditions made worse due to a reduced level or reduced availability of 15-deoxy-prostaglandin J2.

In another aspect, the present invention consists in the use of a compound of formula (IV) as defined above as an agent which enhances the production of 15-deoxy-prostaglandin J2 for the manufacture of a medicament to promote neurite outgrowth or to treat peripheral neuropathy.

The peripheral neuropathy may be due to treatment with chemotherapeutic agents, such as cisplatin, or it may be from other causes, such as diabetic neuropathy.

In a still further aspect, the present invention consists in the use of a compound of formula (IV) as defined above as an agent which enhances the production of 15-deoxy-prostaglandin J2 and in turn activates PPARgamma for the manufacture of a medicament to treat a condition requiring the activation of PPARgamma.

In yet another aspect, the present invention provides the use of a compound of formula (IV) as defined above as an agent which enhances the production of 15-deoxy-prostaglandin J2 for the manufacture of a medicament for the treatment or prophylaxis of cancer.

Still further, the present invention provides the use of a compound of formula (IV) as defined above as an agent which enhances the production of 15-deoxy-prostaglandin J2 for the manufacture of a medicament for inhibiting cancer cell proliferation, inducing apoptosis in cancer cells or inhibiting tumour growth and progression.

Cancer cells to which this aspect of the present invention is particularly useful include colorectal, gastric, breast, hepatic, prostate, bladder, thyroid papillary and oesophageal cancer cells.

Additionally, the compounds of the present invention may be used in the treatment and prophylaxis of:
pain associated with inflammation;
peripheral arterial diseases (comprising disorders that compromise blood flow to the limbs and are caused by peripheral atherosclerosis), and their sequelae such as critical limb ischaemia;
coronary artery disease and its sequelae, such as ischaemic heart disease (e.g. stable and unstable angina) and myocardial infarction (MI);
cerebrovascular diseases and its sequelae such as stroke and transient ischaemic attacks (TIA); liver and kidney ischaemia, e.g. atherosclerotic renal artery stenosis;
metabolic diseases such as type 2 diabetes and its sequelae, such as peripheral arterial diseases, coronary artery disease, vascular diseases of the kidney and diabetic neuropathy;
obesity and its sequela such as type 2 diabetes, peripheral arterial diseases and coronary artery disease;
inflammatory airways diseases such as asthma and chronic obstructive pulmonary disease (COPD), e.g. chronic bronchitis;
chronic neurodegenerative diseases such as Alzheimer's disease, Parkinson's disease, multiple sclerosis and peripheral neuropathies;
acute neurological degenerative conditions such as traumatic brain injury and spinal cord injury;
inflammatory bowel disease;
inflammatory diseases characterised by degeneration of articular cartilage such as rheumatoid arthritis and primary and secondary osteoarthritis and their sequelae; and
wound healing; and
toxicity or peripheral neuropathies caused by chemotherapeutic agents.

Preferably these agents can be used to treat diabetes mellitus and its sequelae; ischemic vascular diseases; pain associated with inflammation; inflammatory skin conditions; spinal cord injury; peripheral neuropathy; multiple sclerosis; inflammatory bowel disease; rheumatoid arthritis, metabolic syndrome X, obesity, acromegaly and wound healing.

Such agents may also be useful in the treatment or prophylaxis of conditions such as inflammatory diseases of peripheral organs, for example the liver and kidneys, as caused by, for example, liver and renal ischaemia.

Other inflammatory conditions that can be treated by these agents include inflammatory airway diseases, such as asthma, rhinitis, bronchitis and chronic obstructive pulmonary disease (COPD).

The invention is illustrated by the accompanying drawings, in which, and hereafter, 7β-hydroxy-EPIAndrosterone is hereinafter referred to as 7β-OH EPIA. In the drawings:
Figure 1 shows the mean percentage cell death combined data from 4 separate experiments in Example 1, with the data expressed as mean±sem;
Figure 2 shows the mean percentage cell death in separate experiments reported in Example 1;
Figure 3 shows the mean percentage cell death in separate experiments reported in Example 1;
Figure 4 shows the mean percentage cell death in separate experiments reported in Example 1;
Figures 5(a) and 5(b) show the effect of increasing concentrations of 7β-OH-EPIA on PGD₂ levels detected in the cell supernatants of peripheral blood mononuclear incubated with 7β-OH-EPIA, in the presence and absence of TNF-α, as reported in Example 2;
Figures 6(a) and 6(b) show the effect of increasing concentrations of 7β-OH-EPIA on PGE₂ levels detected in the cell supernatants of peripheral blood mononuclear
incubated with 7β-OH-EPIA, in the presence and absence of TNF-α, as reported in Example 2;
Figures 7(a) and 7(b) show the effect of increasing concentrations of 7β-OH-EPIA on 15d-PGJ₂ levels detected in the cell supernatants of peripheral blood mononuclear incubated with 7β-OH-EPIA, in the presence and absence of TNF-α, as reported in Example 2;
Figure 8 shows the effects of 7β-hydroxy-EPIA treatment on (A) myeloperoxidase (MPO) and oxidative stress markers namely (B) Prot CO, (C) Tbars and (D) the antioxidant marker GSH in colonic tissue, as described in more detail in Example 4.
Figure 9 shows colonic 15d-PGJ₂ level (A) and quantification of relative mRNA expression of COX-2, mPGES-1 and H-PGDS (B) at various times during 7β-hydroxy-EPIA treatment in Example 4.
Figure 10 shows the effect of 7β-hydroxy-EPIA on colonic synthesis of prostaglandin E₂, D₂ and 15d-PGJ₂ during DSS administration in Example 4.
Figure 11 shows colonic expression of COX-2, mPGES-1 and H-PGDS mRNA during colitis induction in Example 4.

The compounds which may be used in the present invention are those compounds of formula (IV): 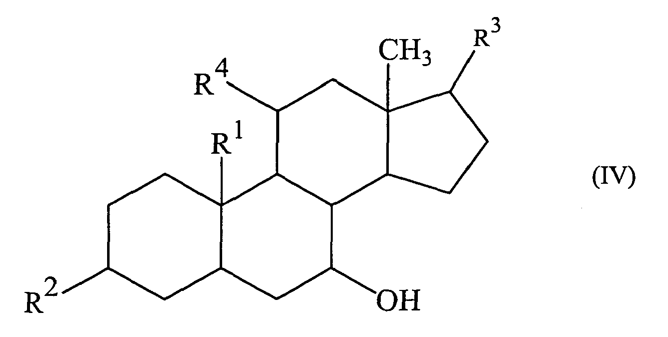 (in which R¹, R², R³ and R⁴ are as defined above) and esters thereof.

Examples of compounds of formula (IV) include 7-hydroxyEPIAndrosterone, which may be in the form of its 7α or 7β isomers, and esters thereof. 7β-hydroxy-EPIAndrosterone, hereinafter referred to as 7β-OH EPIA, has the formula (IVa): 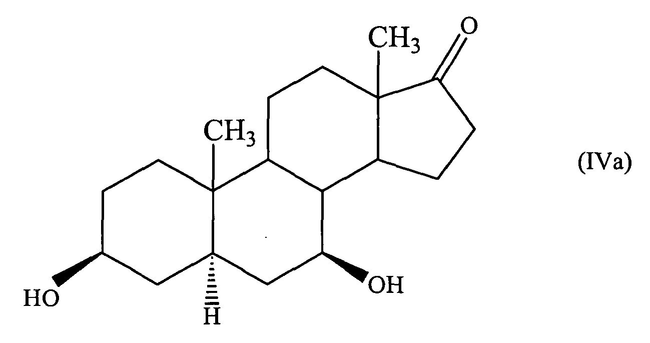 while 7α- hydroxyEPIAndrosterone, hereinafter referred to as 7α-OH EPIA, has the formula (IVb):

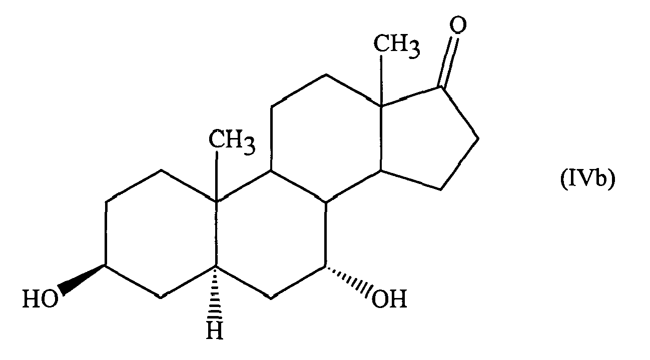

In the compounds of the present invention, where R², R³ or R⁴ represents a halogen atom, this may be a fluorine, chlorine, bromine or iodine atom, preferably a chlorine atom.

Where R², R³ or R⁴ represents an alkoxy group, this may be a straight or branched chain group, preferably having from 1 to 6 carbon atoms. Examples of such groups include the methoxy, ethoxy, propoxy, isopropoxy, butoxy, sec-butoxy, isobutoxy, t-butoxy, pentyloxy and hexyloxy groups.

Where R², R³ or R⁴ represents an aryloxy group, this is preferably a phenoxy or naphthyloxy group.

Where R², R³ or R⁴ represents an acyl group, this may be, for example, an aliphatic acyl group or an aromatic acyl group. Examples of aliphatic acyl groups include groups having from 1 to 6 carbon atoms, such as the formyl, acetyl, propionyl, butyryl, valeryl, isovaleryl, pivaloyl and hexanoyl groups. Examples of aromatic acyl groups include the benzoyl, naphthoyl and toluoyl groups.

It will be appreciated that, where the compound contains a group of formula -

OR, where R is any of the groups and atoms defined above in relation to R² etc., the active species is likely to be the compound containing the free hydroxy group. Accordingly, any group that can be converted *in vivo* to a hydroxy group may be used in place of the hydroxy group.

These compounds may be prepared by a variety of processes, well known in themselves, starting from the parent steroids. For example, they may be prepared by the methods described in EP 1294382.

The compounds of formula (IV) are disclosed in EP 1294382, WO2002/000224 and WO2002/000225 for use in the treatment or prophylaxis of chronic and acute neurodegenerative diseases or conditions and in WO2002/015791 for use in the treatment or prophylaxis of chronic and acute cardiodegenerative diseases or conditions and, of course, such treatment or prophylaxis is excluded from the present claims.

When the compounds of the present invention contain a carboxy group, the compounds of the invention can form esters, which may be prepared by conventional esterification techniques. There is no particular restriction on the nature of the ester, provided that, where the resulting compound is to be used medically, the compound is pharmaceutically acceptable, that is it is not less active, or unacceptably less active, nor more toxic, or unacceptably more toxic, than the parent compound. However, where the compound is to be used for non-medical uses, e.g. as an intermediate in the preparation of other compounds, even this restriction does not apply, and there is then no restriction on the nature of the esters which may be formed.

Examples of ester groups include:
alkyl groups having from 1 to 20 carbon atoms, more preferably from 1 to 10 carbon atoms, such as those exemplified in relation to R⁷, R⁹, R¹¹ or R¹² and higher alkyl groups as are well known in the art, such as the dodecyl, tridecyl, pentadecyl, octadecyl, nonadecyl and icosyl groups;
cycloalkyl groups having from 3 to 7 carbon atoms, for example the cyclopropyl, cyclobutyl, cyclopentyl, cyclohexyl and cycloheptyl groups;
aralkyl groups, in which the alkyl part has from 1 to 3 carbon atoms and the aryl part is a carbocyclic aromatic group having from 6 to 14 carbon atoms, which may be substituted or unsubstituted; examples of such aralkyl groups include the benzyl, phenethyl, 1-phenylethyl, 3-phenylpropyl, 2-phenylpropyl, 1-naphthylmethyl, 2-naphthylmethyl, 2-(1-naphthyl)ethyl, 2-(2-naphthyl)ethyl, benzhydryl (i.e. diphenylmethyl), triphenylmethyl, bis(o-nitrophenyl)methyl, 9-anthrylmethyl, 2,4,6-trimethylbenzyl, 4-bromobenzyl, 2-nitrobenzyl, 4-nitrobenzyl, 3-nitrobenzyl, 4-methoxybenzyl and piperonyl groups;
alkenyl groups having from 2 to 6 carbon atoms, such as the vinyl, allyl, 2-methylallyl, 1-propenyl and isopropenyl groups;
halogenated alkyl groups having from 1 to 6, preferably from 1 to 4, carbon atoms, such as the 2,2,2-trichloroethyl, 2-haloethyl (e.g. 2-chloroethyl, 2-fluoroethyl, 2-bromoethyl or 2-iodoethyl), 2,2-dibromoethyl and 2,2,2-tribromoethyl groups;
substituted silylalkyl groups, for example the 2-tri(C₁ - C₄)alkylsilylethyl groups, especially a 2-trimethylsilylethyl group;
substituted and unsubstituted phenyl groups, for example the phenyl, tolyl and benzamidophenyl groups;
substituted and unsubstituted phenacyl groups, for example the phenacyl group itself or the p-bromophenacyl group;
cyclic and acyclic terpenyl groups, for example the geranyl, neryl, linalyl, phytyl, menthyl (especially m- and p- menthyl), thujyl, caryl, pinanyl, bornyl, norcaryl, norpinanyl, norbornyl, menthenyl, camphenyl and norbornenyl groups;
alkoxymethyl groups, in which the alkoxy part has from 1 to 6, preferably from 1 to 4, carbon atoms and may itself be substituted by a single unsubstituted alkoxy group, such as the methoxymethyl, ethoxymethyl, propoxymethyl, isopropoxymethyl, butoxymethyl and methoxyethoxymethyl groups;
aliphatic acyloxyalkyl groups, in which the acyl group is preferably an alkanoyl group and is more preferably an alkanoyl group having from 2 to 6 carbon atoms, and the alkyl part has from 1 to 6, and preferably from 1 to 4, carbon atoms such as the acetoxymethyl, propionyloxymethyl, butyryloxymethyl, isobutyryloxymethyl, pivaloyloxymethyl, 1-pivaloyloxyethyl, 1-acetoxyethyl, 1-isobutyryloxyethyl, 1-pivaloyloxypropyl, 2-methyl-1-pivaloyloxypropyl, 2-pivaloyloxypropyl, 1-isobutyryloxyethyl, 1-isobutyryloxypropyl, 1-acetoxypropyl, 1-acetoxy-2-methylpropyl, 1-propionyloxyethyl, 1-propionyloxypropyl, 2-acetoxypropyl and 1-butyryloxyethyl groups;
cycloalkyl-substituted aliphatic acyloxyalkyl groups, in which the acyl group is preferably an alkanoyl group and is more preferably an alkanoyl group having from 2 to 6 carbon atoms, the cycloalkyl substituent has from 3 to 7 carbon atoms, and the alkyl part has from 1 to 6, preferably from 1 to 4, carbon atoms, such as the cyclohexylacetoxymethyl, 1-(cyclohexylacetoxy)ethyl, 1-(cyclohexylacetoxy)propyl, 2-methyl-1-(cyclohexylacetoxy)propyl, cyclopentylacetoxymethyl, 1-(cyclopentylacetoxy)ethyl, 1-(cyclopentylacetoxy)propyl and 2-methyl-1-(cyclopentylacetoxy)-propyl, groups;
alkoxycarbonyloxyalkyl groups, especially 1-(alkoxycarbonyloxy)ethyl groups, such as the 1-methoxycarbonyloxyethyl, 1-ethoxycarbonyloxyethyl, 1-propoxycarbonyloxyethyl, 1-isopropoxycarbonyloxyethyl, 1-butoxycarbonyloxyethyl, 1-isobutoxycarbonyloxyethyl, 1-sec-butoxycarbonyloxyethyl, 1-t-butoxycarbonyloxyethyl, 1-(1-ethylpropoxycarbonyloxy)ethyl and 1-(1,1-dipropylbutoxycarbonyloxy)ethyl groups, and other alkoxycarbonylalkyl groups, in which both the alkoxy and alkyl groups have from 1 to 6, preferably from 1 to 4, carbon atoms, such as the 2-methyl-1-(isopropoxycarbonyloxy)propyl, 2-(isopropoxycarbonyloxy)propyl, isopropoxycarbonyloxymethyl, t-butoxycarbonyloxymethyl, methoxycarbonyloxymethyl and ethoxycarbonyloxymethyl groups;
cycloalkylcarbonyloxyalkyl and cycloalkyloxycarbonyloxyalkyl groups, for example the 1-methylcyclohexylcarbonyloxymethyl, 1-methylcyclohexyloxycarbonyloxymethyl, cyclopentyloxycarbonyloxymethyl, cyclopentylcarbonyloxymethyl, 1-(cyclohexyloxycarbonyloxy)ethyl, 1-(cyclohexylcarbonyloxy)ethyl, 1-(cyclopentyloxycarbonyloxy)-ethyl, 1-(cyclopentylcarbonyloxy)ethyl, 1-(cycloheptyloxycarbonyloxy)ethyl, 1-(cycloheptylcarbonyloxy)ethyl, 1-methylcyclopentylcarbonyloxymethyl, 1-methylcyclopentyloxycarbonyloxymethyl, 2-methyl-1-(1-methylcyclohexylcarbonyloxy)-propyl, 1-(1-methylcyclohexylearbonyloxy)propyl, 2-(1-methylcyclohexylcarbonyloxy)propyl, 1-(cyclohexylcarbonyloxy)propyl, 2-(cyclohexylcarbonyloxy)propyl, 2-methyl-1-(1-methylcyclopentylcarbonyloxy)propyl, 1-(1-methylcyclopentylcarbonyloxy)propyl, 2-(1-methylcyclopentylcarbonyloxy)propyl, 1-(cyclopentylcarbonyloxy)propyl, 2-(cyclopentylcarbonyloxy)propyl, 1-(1-methylcyclopentylcarbonyloxy)ethyl, 1-(1-methylcyclopentylcarbonyloxy)propyl, adamantyloxycarbonyloxymethyl, adamantylcarbonyloxymethyl, 1-adamantyloxycarbonyloxyethyl and 1-adamantylcarbonyloxyethyl groups;
cycloalkylalkoxycarbonyloxyalkyl groups, for example the cyclopropylmethoxycarbonyloxymethyl, cyclobutylmethoxycarbonyloxymethyl, cyclopentylmethoxycarbonyloxymethyl, cyclohexylmethoxycarbonyloxymethyl, 1-(cyclopropylmethoxycarbonyloxy)ethyl, 1-(cyclobutylmethoxycarbonyloxy)ethyl, 1-(cyclopentylmethoxycarbonyloxy)ethyl and 1-(cyclohexylmethoxycarbonyloxy)ethyl groups;
terpenylcarbonyloxyalkyl and terpenyloxycarbonyloxyalkyl groups, for example the 1-(menthyloxycarbonyloxy)ethyl, 1-(menthylcarbonyloxy)ethyl, menthyloxycarbonyloxymethyl, menthylcarbonyloxymethyl, 1-(3-pinanyloxycarbonyloxy)ethyl, 1-(3-pinanylcarbonyloxy)ethyl, 3-pinanyloxycarbonyloxymethyl and 3-pinanylcarbonyloxymethyl groups; 5-alkyl- or 5-phenyl- (2-oxo-1,3-dioxolen-4-yl)alkyl groups, for example the (5-methyl-2-oxo-1,3-dioxolen-4-yl)methyl, (5-phenyl-2-oxo-1,3-dioxolen-4-yl)methyl, (5-isopropyl-2-oxo-1,3-dioxolen-4-yl)methyl, (5-t-butyl-2-oxo-1,3-dioxolen-4-yl)methyl and 1-(5-methyl-2-oxo-1,3-dioxolen-4-yl)ethyl groups; and
other groups, especially groups which are easily removed *in vivo* such as the phthalidyl, indanyl and 2-oxo-4,5,6,7-tetrahydro-1,3-benzodioxolen-4-yl groups.

Also, if the compounds of the present invention contain a carboxy group, they can be converted into salts with a base by conventional methods. There is no particular restriction on the nature of such salts, provided that, where the compounds are to be used medically, the compounds are pharmaceutically acceptable. However, where the compound is to be used for non-medical uses, e.g. as an intermediate in the preparation of other compounds, even this restriction does not apply, and there is then no restriction on the nature of the salts which may be formed. Examples of such salts include: salts with an alkali metal, such as sodium, potassium or lithium; salts with an alkaline earth metal, such as barium or calcium; salts with another metal, such as magnesium or aluminium; ammonium salts; organic base salts, such as a salt with methylamine, dimethylamine, triethylamine, diisopropylamine, cyclohexylamine or dicyclohexylamine; and salts with a basic amino acid, such as lysine or arginine. We prefer the pharmaceutically acceptable salts.

The compounds of the present invention can also be converted to salts with acids by conventional methods. There is no particular restriction on the nature of such salts, provided that, where the compounds are to be used medically, the compounds are pharmaceutically acceptable. However, where the compound is to be used for non-medical uses, e.g. as an intermediate in the preparation of other compounds, even this restriction does not apply, and there is then no restriction on the nature of the salts which may be formed. Examples of such salts include: salts with mineral acids, especially hydrohalic acids (such as hydrofluoric acid, hydrobromic acid, hydroiodic acid or hydrochloric acid), nitric acid, perchloric acid, carbonic acid, sulphuric acid or phosphoric acid; salts with lower alkylsulphonic acids, such as methanesulphonic acid, trifluoromethanesulphonic acid or ethanesulphonic acid; salts with arylsulphonic acids, such as benzenesulphonic acid or p-toluenesulphonic acid; salts with organic carboxylic acids, such as acetic acid, fumaric acid, tartaric acid, oxalic acid, maleic acid, malic acid, succinic acid, benzoic acid, mandelic acid, ascorbic acid, lactic acid, gluconic acid or citric acid; and salts with amino acids, such as glutamic acid or aspartic acid. We prefer the pharmaceutically acceptable salts.

The compounds of the present invention may therefore be used in the treatment or prophylaxis of a variety of chronic and acute diseases or conditions, and, for these purposes, may be formulated as conventional pharmaceutical preparations, as is well known in the art. Thus, the compounds may be administered orally, e.g. in the form of tablets, capsules, granules, powders, syrups, sprays or other such well known forms, or parenterally, e.g. by injections, sprays, eye drops, adhesive plasters or suppositories, etc.

These pharmaceutical preparations can be prepared by conventional means and may contain known adjuvants of a type commonly used in this field, for example vehicles, binders, disintegrators, lubricants, stabilizers, corrigents, etc. depending upon the intended use and form of the preparation. The dose will depend upon the condition, age, and body weight of the patient as well as upon the nature and severity of the disorder to be treated, but in the case of oral administration to an adult human patient, we would normally suggest a total daily dose of from 0.01 to 50 mg/kg body weight (more preferably from 0.05 to 20 mg/kg body weight), which may be administered in a single dose or in divided doses, e.g. from one to three times a day.

In general, the compounds of the present invention may be used for the treatment or prophylaxis of a variety of inflammatory conditions or conditions arising from the metabolic pathway(s) which lead to inflammation. Examples of uses to which these compounds may be put include: promotion of neurite growth; treatment or prophylaxis of diabetes mellitus (especially Type 2 diabetes mellitus) and its sequelae; treatment and prophylaxis of ischemic vascular diseases; treatment of pain associated with inflammation; and treatment and prophylaxis of inflammatory skin conditions, including psoriasis and wound healing. Other inflammatory conditions which may be treated or prevented, or whose effects may be mitigated, by use of the compounds of the present invention include spinal cord injury, peripheral neuropathy, multiple sclerosis, inflammatory bowel disease, rheumatoid arthritis; and peripheral neuropathy or toxicity caused by chemotherapeutic agents such as cisplatin, or other causes, such as diabetic neuropathy. Finally, conditions which may be treated or prevented, or whose effects may be mitigated, by use of the compounds of the present invention also include various types of cancer.

### The invention is further illustrated by the following non-limiting Examples.

### EXAMPLE 1

### Protective effects of 7β-hydroxy-EPIAndrosterone against ischaemia in PC-12 cells: inhibition by the cyclooxygenase (COX) inhibitor indomethacin

The purpose of this Example was to investigate whether 7β-OH-EPIA retains its neuroprotective efficacy in a model of hypoxia when prostaglandin synthesis is attenuated by indomethacin. The main experimental system used was ischaemia-induced cytotoxicity in PC12 cells. The experimental end point measured was cell death.

Ischaemia-induced PC-12 cell death was consistently reduced by 7β-OH-EPIA. Indomethacin (10µM), which blocks prostaglandin synthesis, had no direct effect on ischemia-induced cell death, but fully antagonised the neuroprotective effects of 1µM and 10µM 7β-OH-EPIA, supporting the hypothesis that prostaglandin synthesis is required for the neuroprotective effect of 7β-hydroxy-EPIA.

### Methodology

### PC-12 cell culture

PC-12 cells were maintained on flasks coated with Collagen type1 in PC-12 media with the following composition; RPMI 1640 without L-glutamine with 2mM L-glutamine, 10mM HEPES, 1mM Sodium Pyruvate, additional glucose to give a final concentration of 4.5g/L (RPMI normally has 2g/L), 10% heat inactivated horse serum, 5% foetal calf serum and 50 units of penicillin/streptomycin. Media was changed every 2 days.

### PC-12 cell assays

Confluent cultures of PC-12 cells were differentiated by culturing for 7 days in PC-12 medium without serum but with 50ng/ml NGF (PC-12 NGF media). The cells were harvested, washed, counted and 1 x 10⁵ PC-12 cells/well were plated overnight in a micro titre plate in glucose free PC-12 NGF medium. The medium was then changed to glucose-free PC-12 NGF medium containing the compounds to be tested and the plates left under normoxic conditions for 30 minutes.

During this stage, the media and test substances for the anoxic exposure were placed in a chamber and de-oxygenated with 95%N₂/5%CO₂. The media in the plates was changed with the de-oxygenated media and the plates then placed in an anaerobic chamber gassed with 95%N₂ 5%CO₂ for 10 minutes before being sealed and incubated at 37°C overnight (18 hours). For normoxia controls, the cells were treated identically to ischemic treatments except that all incubations were in 5%CO₂/95% air.

Viability was determined using Trypan Blue exclusion.

### Results

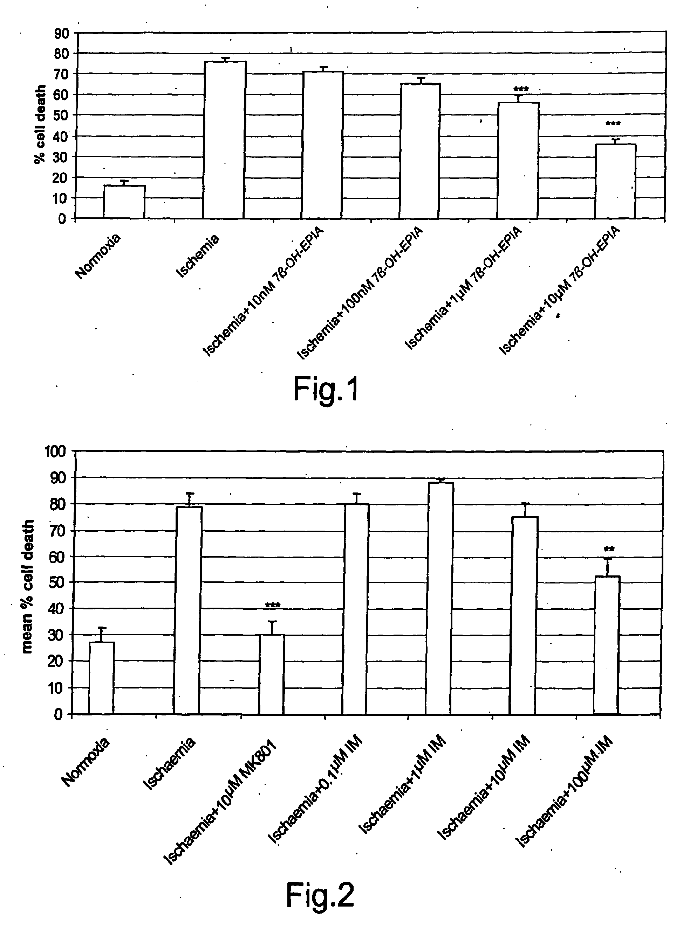

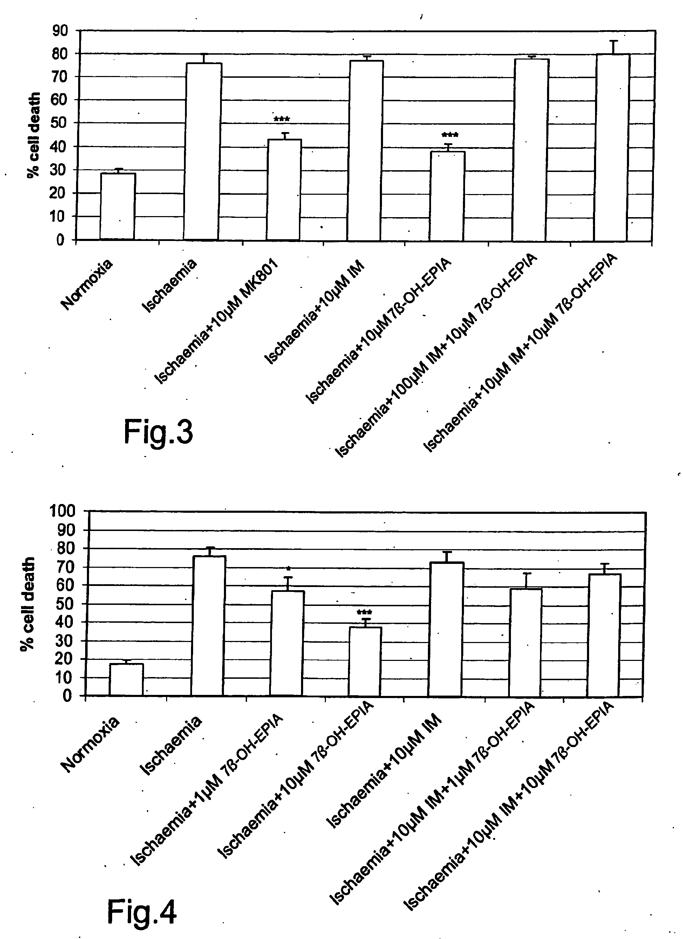

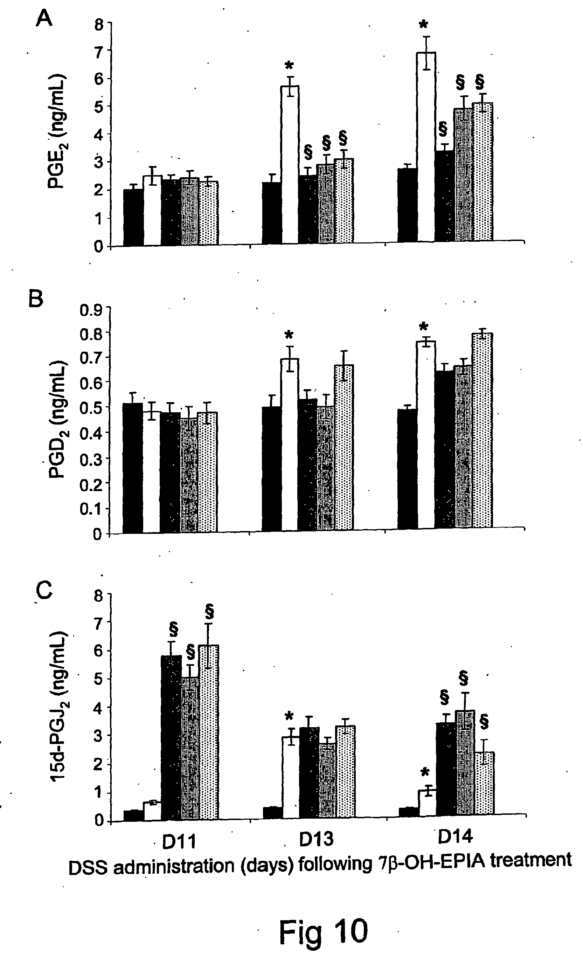

PC-12 cells were relatively resistant to hypoxia. Because of this, we used a more severe combined oxygen-glucose deprivation (ischaemia) protocol to initiate toxicity (Figure 1). 7β-OH-EPIA was dose-dependently cytoprotective in this assay with significant neuroprotection being observed at 1µM (26% decrease in cell death) and 10µM (53% decrease in cell death). Figure 1 presents combined data from 4 experiments showing this effect. Figure 1 shows the mean percentage cell death from combined data from 4 separate experiments. The data is expressed as mean±sem. 76%±2.2% cell death was observed with ischemia alone. Statistically significant decrease in cell death was observed with 1 µM 7β-OH-EPIA (26% decrease in cell death, p<0.001), 10µM 7β-OH-EPIA (53% decrease in cell death, p<0.001),
* **=p<0.001 vs. ischemia alone.
   The NMDA receptor antagonist MK-801 also reduced ischaemia-induced toxicity of PC-12 cells (Figure 2). The cyclooxygenase inhibitor indomethacin had a moderate protective effect at 100µM (29% reduction in cell death), but not at lower concentrations (Figure 2). Figure 2 shows the mean percentage cell death. The data is expressed as mean±sem. 79%±5.3% cell death was observed with ischemia alone. Statistically significant decrease in cell death was observed with 100µM Indomethacin (IM) (29% decrease in cell death, p<0.05), 10µM MK801 (62% decrease in cell death, p<0.001), **= p<0.01, ***=p<0.001 vs. ischemia alone.
   The neuroprotective effect of 7β-OH-EPIA was fully antagonised by 10-100µM indomethacin (IM) (Figure3) with toxicity in these cultures being indistinguishable from cultures exposed to ischaemia alone. Figure 3 shows the mean percentage cell death. The data is expressed as mean±sem. 76%±4.2% cell death was observed with ischaemia alone. A statistically significant decrease in cell death was observed with 10µM 7β-OH-EPIA (50% decrease in cell death, p<0.001), 10µM MK801 (42% decrease in cell death, p<0.001), ***=p<0.001 vs. ischemia alone.
   As seen in Figure 4, 10µM indomethacin fully antagonised the protective effect of both 1µM and 10µM 7β-OH-EPIA. Figure 4 shows the mean percentage cell death. The data is expressed as mean±sem. 76%±4.8% cell death was observed with ischaemia alone. A statistically significant decrease in cell death was observed with 1µM 7β-OH-EPIA (25% decrease in cell death, p<0.05), 10µM 7β-OH-EPIA (50% decrease in cell death, p<0.001), *= p<0.05, ***=p<0.001 vs. ischemia alone.

### Conclusions

Ischemia-induced PC-12 cell death was consistently reduced by 7β-OH-EPIA. Indomethacin, which blocks prostaglandin synthesis, did not directly influence PC-12 cell death at concentrations up to 10µM. However, when the concentration was increased further to 100µM a moderate neuroprotective effect was observed.

Indomethacin (10µM) had no direct effect on ischemia-induced cell death, but significantly attenuated the neuroprotective effect of 7β -OH-EPIA, supporting the hypothesis that prostaglandin synthesis is required for the neuroprotective effect of 7 β-OH-EPIA.

### EXAMPLE 2

### The effect of 7β-hydroxy-EPIAndrosterone on the production of Prostaglandins D₂, E₂ and 15-deoxy-A^{12,14}- J₂ by human mononuclear cells

The purpose of this Example was to ascertain whether 7β-OH-EPIA could induce the biosynthesis of specific metabolites of arachidonic acid in human monocytic cells namely, prostaglandin D₂ (PGD₂), prostaglandin E₂ (PGE₂) and 15-deoxy-Δ^{12,14}-prostaglandin J₂ (15d-PGJ₂).

Monocytic blood cells were exposed to a range of concentrations of 7 β-OH-EPIA in either the absence or presence of Tumour Necrosis Factor- (TNF-α), a pro-inflammatory stimulus, and the amount of PGD₂, PGE₂ and 15d-PGJ₂ produced was measured by enzyme immunoassay (EIA).

7β-OH-EPIA (0.1nM-1000nM) induced a concentration-dependant increase in the production of PGD₂ from normal human peripheral blood mononuclear cells. TNF-α increased PGD₂ production compared to control and this was enhanced at the highest concentration of 7 β -OH-EPIA. 7β-OH-EPIA (1nm-1000nM) appeared to have no significant effect on PGE₂ biosynthesis in normal human peripheral blood mononuclear cells but completely suppressed TNF-αinduced increases in PGE₂ production. 7β-OH-EPIA (0.1nM-1000nM) was found to increase 15d-PGJ₂ production in normal human peripheral blood mononuclear cells by approximately 9-12-fold in the absence of TNF-α and by 2-2.5-fold in the presence of TNF-α compared to their respective controls.

### Methodology

### Peripheral Blood Mononuclear Cells

Mononuclear cells (monocytes and lymphocytes) were prepared by subjecting 48 ml of whole human blood to Ficoll/hypaque density centrifugation. Blood was layered on top of the ficoll (density = 1.077 g/ ml) and centrifuged at 400 g for 1 hour (22°C), after which cells at the interphase were removed by pipette and transferred to fresh tubes. Tubes were filled with RPMI 1640 culture medium (2 volumes) thoroughly mixed and then centrifuged at 400 g for 5 minutes (22°C). The supernatant was discarded and the cell pellet was re-suspended in RPMI 1640 culture medium and the volume adjusted to give the appropriate number of cells per incubation as indicated in the results section below. Cells were incubated in sterile plastic 1.5 ml tubes in a final volume of 1 ml RPMI 1640 medium for 18 hours at 37°C , 5 % CO₂ in air and 100 % humidity. 7β-OH-EPIA was then added and cells incubated for a further hour at 37°C before adding recombinant human Tumour Necrosis Factor-α (TNF-α) and incubations continued for a further 3 hours. 7β-OH-EPIA was prepared in dimethyl sulphoxide (DMSO), all remaining agents were prepared in RPMI 1640 medium. The requisite controls contained either medium or the same concentration of DMSO which was always < 0.1 % v/v. Incubations were terminated by centrifuging tubes at 11,000g for 30 seconds at 22 °C and transferring the supernatants to fresh 1.5 ml tubes. Samples were then either processed immediately for PGD₂ estimation as described below or stored at - 20 °C prior to PGE₂ or 15d-PGJ₂ measurement.

### Prostaglandin Enzyme Immunoassays (EIAs)

Prostaglandin production by human monocytic cells in response to 7β-OH-EPIA in either the absence or presence of stimulation with TNF-α was determined by measuring the extracellular levels of eicosanoid using commercially available EIA kits.

### PGD₂ EIA

PGD₂ cannot be assayed directly from cell supernatants because it is chemically unstable and rapidly degrades to a number of J series prostaglandins including, PGJ₂, Δ12-PGJ₂, and 15-deoxy-Δ^{12,14}-PGJ2. In order to circumvent this problem, unstable PGD₂ was chemically treated to give a stable derivative, in this case prostaglandin D₂-Methoxamine (PGD₂-MOX) which was be stored for subsequent analysis. Immediately following termination of incubations, 100 µl of sample supernatant was added to 1.5 ml tubes containing 100 µl of a Methyl Oximating Reagent (methoxylamine hydrochloride (MOX-HCl) and sodium acetate dissolved in a 10:90 v/v, ethanol/water solution. Tubes were then placed in a water bath and the reaction allowed to proceed for 30 minutes at 60 °C. At the end of this period samples were stored at - 80 °C. Levels of PGD₂ were subsequently estimated using Cayman Chemical's Prostaglandin D₂-MOX EIA Kit (Cat. No. 512011).

### PGE₂ EIA

Extracellular levels of PGE₂ were estimated using R & D Systems' Parameter PGE₂ EIA Kit according to the manufacturer's instructions for the high sensitivity protocol.

### 15d-PG₂ EIA

Extracellular levels of 15d-PGJ₂ were estimated using Assay Designs' Correlate-EIA Prostaglandin 15-deoxy-Δ^{12,14}-prostaglandin J₂ EIA Kit (Cat. No.900-023) according to the manufacturer's instructions.

### Statistical Analysis

Results are expressed as the mean± s.d. for n = 3 incubations. An unpaired Students *t*-Test was used to determine the probability (P) that two sets of data were different from each other. The difference was considered to be significant when P < 0.05. Statistical calculations were carried out on an Apple Macintosh computer using the software package Statview, from Abacus Concepts Inc.

### Results

### Effect of 7β-OH-EPIA on PGD₂ production by human mononuclear cells

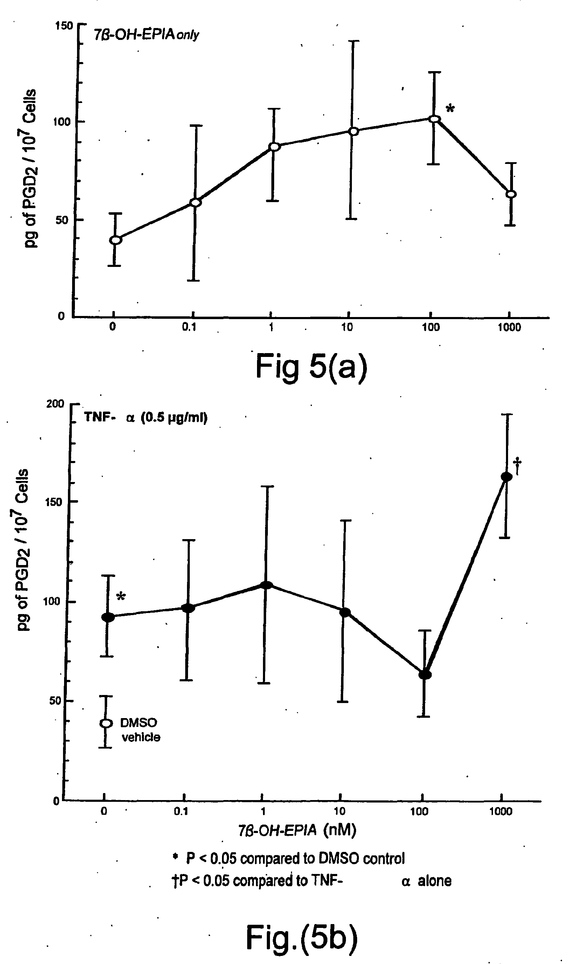

Figure 5(a) shows the effect of increasing concentrations of 7β-OH-EPIA (0.1 nM - 1000 nM) on PGD₂ levels detected in the cell supernatants of 1 x 10⁷ peripheral blood mononuclear cells/ml incubated for 4 hours with 7β-OH-EPIA. 7 β -OH-EPIA appeared to induce a concentration dependant increase in PGD₂ production reaching a maximum at 100 nM 7 β -OH-EPIA (102 ± 24 pg/ml PGD₂) which was significantly greater than control (40 ± 13 pg/ml PGD₂; P = 0.01).
Figure 5(b) shows the effect of increasing concentrations of 7 β -OH-EPIA (0.1 nM - 1000 nM) on PGD₂ levels detected in the cell supernatants of mononuclear cells incubated in the presence of TNF-α (0.5 µg/ml). TNF-α stimulated a 2.3-fold increase in PGD₂ compared to DMSO vehicle control (P < 0.05). At concentrations of 0.1 nM - 100 nM 7 β -OH-EPIA appeared to have no effect on TNF-α-induced PGD₂ biosynthesis. At the highest concentration of 7 β -OH-EPIA used in this experiment (1000 nM) PGD₂ levels increased to 164 ± 31 pg/ml (P = 0.03 compared to TNF-α alone). *Effect of 7β-OH-EPIA on PGE₂ production by human mononuclear cells*

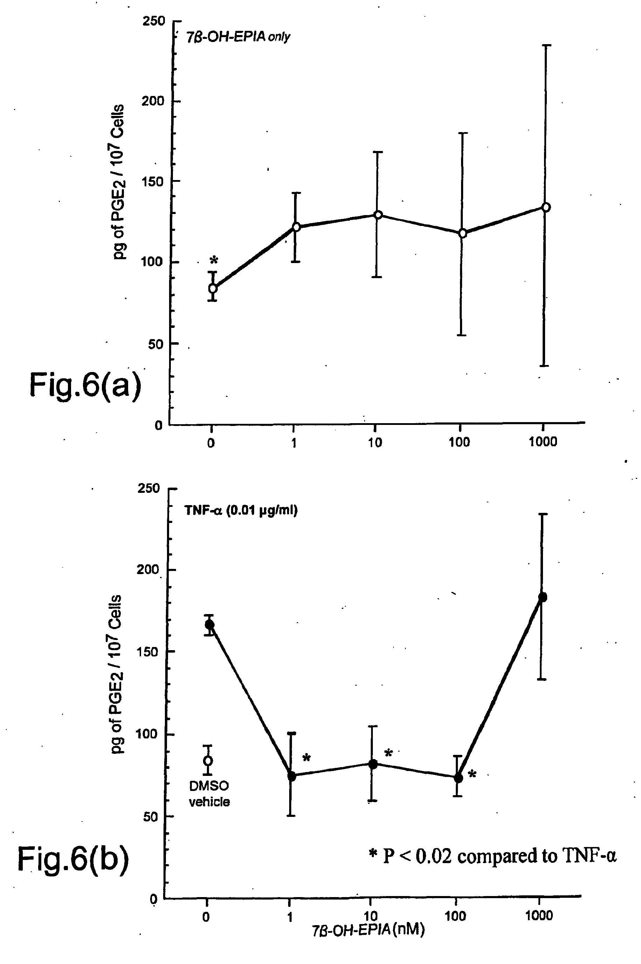

Figure 6(a) shows the effect of increasing concentrations of 7β-OH-EPIA (1 nM - 1000 nM) on PGE₂ levels detected in the cell supernatants of 6 x 10⁵ peripheral blood mononuclear cells/ml incubated for 4 hours with 7β-OH-EPIA. 7β-OH-EPIA appeared to increase PGE₂ levels compared to DMSO controls, however, these increases were not statistically significantly different.
Figure 6(b) shows the effect of increasing concentrations of 7β-OH-EPIA (1 nM - 1000 nM) on PGE₂ levels detected in the cell supernatants of mononuclear cell incubated in the presence of TNF-α (10 ng/ml). TNF-α stimulated a significant 1.97-fold increase in PGE₂ compared to DMSO control (P = 0.001). At concentrations of 1 nM - 100nM, 7 β -OH-EPIA significantly suppressed TNF-α-induced PGE₂ biosynthesis from 167 ± 6 pg/ml in response to TNF-α alone to 75 ± 25 pg/ml, 82 ±23 pg/ml and 74 ± 12 pg/ml for 1 nM, 10 nM and 100 nM 7β-OH-EPIA respectively (all P < 0.02 compared to TNF-α only). At the highest concentration of 7β-OH-EPIA used in this experiment (1000 nM) no effect on TNF-α induced PGE₂ production was observed. *Effect of 7β-OH-EPIA on 15d-PGJ₂ production by human mononuclear cells*

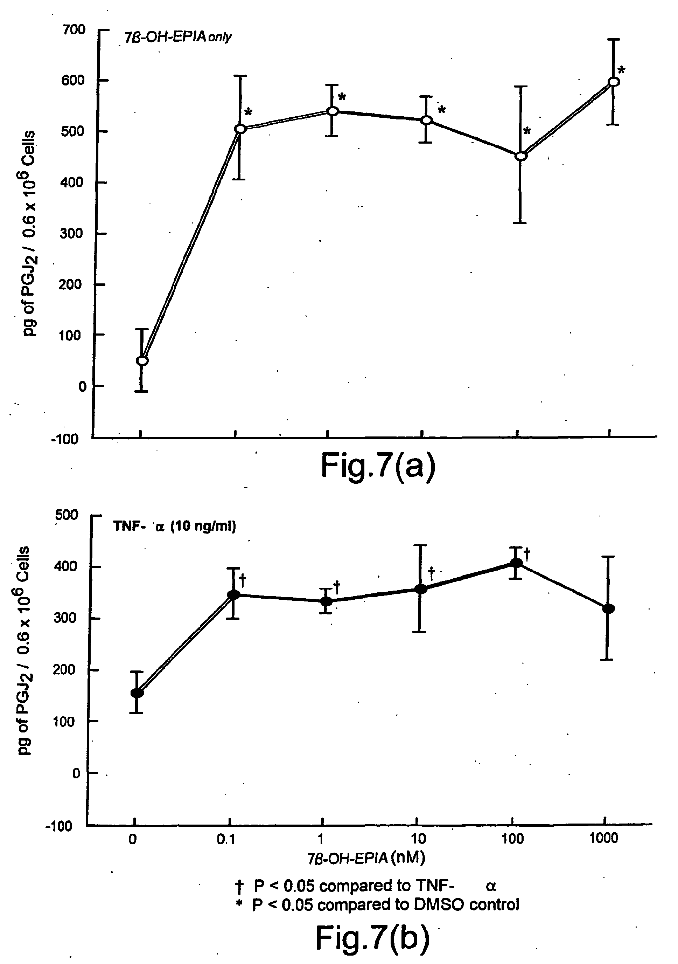

Figure 7(a) shows the effect of increasing concentrations of 7β-OH-EPIA (0.1 nM - 1000 nM) on 15d-PGJ₂ levels detected in the cell supernatants of mononuclear cells incubated for 4 hours with 7β-OH-EPIA. 7β-OH-EPIA significantly increased 15d-PGJ₂ levels by approximately 9-12-fold at all concentrations used. Levels were increased from 51 ± 6 pg/ml for DMSO controls to 506 ±101 pg/ml, 539 ± 51pg/ml, 520 ± 45 pg/ml, 450 ± 133 pg/ml and 590 ± 84 pg/ml for 0.1 nM, 1 nM, 10 nM, 100nM and 1000nM 7β-OH-EPIA respectively (all P < 0.05 compared with DMSO controls).
Figure 7(b) shows the effect of increasing concentrations of 7β-OH-EPIA (0.1 nM - 1000 nM) on 15d-PGJ₂ levels detected in the cell supernatants of mononuclear cells incubated with TNF-α(10 ng/ml). At the concentration of cytokine used in this experiment, TNF-α appeared to stimulate a small increase in 15d-PGJ₂, however, this was not significantly different from DMSO controls. At all concentrations used 7β-OH-EPIA increased 15d-PGJ₂ levels in the presence of TNF-α. 15d-PGJ₂ levels were increased from 157 ± 39 pg/ml in the presence of TNF-α alone to 348 ± 48 pg/ml, 334 ± 24 pg/ml, 356 ± 85 pg/ml, 406 ± 30 pg/ml and 318 ± 100 pg/ml in the presence of TNF-α plus 0.1 nM, 1 nM, 10 nM, 100 nM and 1000 nM 7β-OH-EPIA respectively (all P < 0.05 compared with TNF-α only).

### EXAMPLE 3

7α-hydroxy-DHEA, 7β-hydroxy-DHEA and 7β-hydroxy-EPIA are native metabolites of dehydroepiandrosterone (DHEA) and epiandrosterone (EPIA). Since numerous steroids are reported to interfere with inflammatory and immune processes, our objective was to test the effects of these hydroxysteroids on PG production and related enzyme gene expression. Human peripheral blood monocytes (PBMC) were cultured for 4h and 24 hours in the presence of each of the steroids (1-100 nM), with and without addition of the pro-inflammatory cytokine TNF-α (10 ng/mL). Levels of PGE₂, PGD₂ and 15-deoxy-Δ^{12,14}-PGJ₂ (15d-PGJ₂) were measured in the incubation medium, and cell content of mRNA of cyclooxygenase (COX-2), and PGE synthase (m-PGES1) was assessed by quantitative RT-PCR. Addition of TNF-α resulted in elevated PG production and increased COX-2 and m-PGES1 mRNA levels. Among the three steroids tested, only 7β-hydroxy-EPIA decreased COX-2 and m-PGES1 expression while markedly decreasing PGE₂ and increasing 15d-PGJ₂ production. These results indicate that 7β-hydroxy-EPIA has anti-inflammatory effects.

### 1.1. Human PBMC preparation and culture

Whole blood was collected from donors in EDTA-supplemented pouches at the Etablissement Français du Sang (Brest, France). The PBMC were isolated then from whole blood under sterile conditions within 36 hours after collection. Gradient density centrifugation was carried out on Ficoll (Eurobio). After washing in RPMI 1640 medium (Eurobio), cells were suspended in RPMI 1640 medium supplemented with 10% heat-inactivated fetal calf serum (Eurobio), 2 mM glutamine (D. Dutscher), 100 U of penicillin/ml (D. Dutscher), and 100 µg of streptomycin/ml (D. Dutscher). Monocytes were selected by plastic adherence for 1 h and approximately 10⁷ cells were plated in 6-well tissue culture plates (3 mL medium per well). All incubations were performed in a humidified incubator at 37°C and 5% CO₂. Cells were recovered and dispersed in 2ml fresh incubation medium supplemented with either 7β-hydroxyEPIA, 7β-hydroxy-DHEA, or 7α-hydroxy-DHEA (in 20 µL ethanol) in the presence or absence of 0.01 µg/mL TNF-α (Sigma-Aldrich). Control incubations contained 20µl ethanol but no steroid. Supernatants were collected after 4h and 24 hours incubations for measurement of their PG contents and cells were used for subsequent RNA isolation. A single step extraction method using the Trizol Reagent (Invitrogen, Cergy-Pontoise, France) provided total RNA.

### 1.2. Real time reverse transcriptase PCR

cDNAs were synthesized from TURBO DNase I - treated RNA (Ambion, Huntingdon, UK) using the Superscript first strand synthesis system kit (Invitrogen). RT-PCR amplification mixtures (50µL) contained 2,5x RealMaster Mix/20x SYBR solution (11,25 µL) (Eppendorf, Le Pecq, France) and 200 nM forward and reverse primers. Reactions were run on a RealPlex ep gradient S mastercycler (Eppendorf). The cycling conditions were 10 minutes at 95°C and 45 cycles at 95°C, 55°C and 68°C for 15seconds, 30 seconds and 30 seconds, respectively. Each assay included a standard curve of four serial dilution points of control cDNA. The HPRT1 housekeeping gene was used for quantification. All oligonucleotide primers (Table 2), were synthesized by Genecust/Distribio (Evry, France). The specificity of the amplified product was monitored by examining the melting curve of the product and confirmed by analysis on agarose gel electrophoresis.

### 1.3. PG measurements

Commercially available EIA kits were used for the determination of PGE₂ levels (Oxford Biomedical Research, UK) and 15d-PGJ₂ levels (Assay designs, Euromedex, France) in culture media. Measurement of PGD₂ levels was obtained using the prostaglandin D₂-MOX EIA kit (Cayman Chemical, Euromedex). In this case, and prior to assay, the fresh samples were immediately treated with the MOX-HCl reagent, which converted PGD₂ into PGD₂-MOX, thus preventing any further chemical degradation.

### 1.4. Statistical analysis of data

All assays were carried out in triplicate and results were plotted as mean ± S.E.M. One-way analysis of variance was performed followed by Duncan's multiple range tests in order to compare the differences between groups. Differences were considered statistically significant when p<0.05.

### 2.1. Effects of 7α-hydroxy-DHEA

Human PBMC were cultured with and without addition of TNF-α for either 4 or 24 hours. PGE₂, PGD₂ and 15d-PGJ₂ levels were measured in the culture media and mRNA production of related genes (COX-2, m-PGES1) measured in the cell. The data obtained are shown in Table 2. Absence of TNF-α and supplementation with three different concentrations of 7α-hydroxy-DHEA caused no significant change in PGE₂, PGD₂ and 15d-PGJ₂ levels in cultures for 4hours. Only 15d-PGJ₂ showed a moderate increase after 24 h culture. Incubation with 7α-hydroxy-DHEA significantly increased m-PGES1 mRNA levels in cultures after 24 hours.

The presence of TNF-α caused the expected increase in the levels of all PG after 24 h. Incubation with 7α-hydroxy-DHEA for 4 h did not change PG levels at any of the concentrations tested. In contrast, levels of PGE₂ and of the couple PGD₂-15d-PGJ2 were significantly increased and decreased respectively after co-incubation with 7α-hydroxy-DHEA and TNF-α for 24 h, when compared with TNF-α alone. Furthermore, incubation with TNF-α (i.e. inflammatory activation) led to a marked increase in COX-2 and m-PGES1 mRNA production. Co-incubation with 7α-hydroxy-DHEA caused no consistent changes in mRNA levels when compared with TNF-α alone.

### 2.2. Effects of 7β-hydroxy-DHEA

Human PBMC were cultured with and without addition of TNF-α for either 4 or 24 hours. PGE₂, PGD₂ and 15d-PGJ₂ levels were measured in the culture media and mRNA production of related genes (COX-2, m-PGES1) were measured in the cell. The data obtained are shown in Table 3. Incubation with three concentrations of 7β-hydroxy-DHEA for 4hours in the absence of TNF-α caused no significant changes in PGE₂, PGD₂ and 15d-PGJ₂ levels in the culture medium. However, PGD₂ and 15d-PGJ₂, as well as m-PGES1 mRNA levels were increased after 24 hours of incubation with 7β-hydroxy-DHEA in culture.

The presence of TNF-α caused the expected increase in all PG at 24h. Co-incubation with 7β-hydroxy-DHEA for 4 hours did not further change PG levels at any of the concentrations tested. In contrast, both PGD₂ and 15d-PGJ₂ levels were significantly decreased after co-incubation with 7β-hydroxy-DHEA and TNF-α for 24 h, when compared with TNF-α alone. Furthermore, incubation with TNF-α (i.e. inflammatory activation) led to a marked increase in COX-2 and m-PGES1 mRNA production. Co-incubation with 7β-hydroxy-DHEA caused no consistent changes in mRNA levels when compared with TNF-α alone.

### 2.3. Effects of 7β-hydroxy-EPLA

Human PBMC were cultured with and without addition of TNF-α for either 4 or 24 hours. PGE₂, PGD₂ and 15d-PGJ₂ levels were measured in the culture media and mRNA production of related genes (COX-2, m-PGES1) were measured in the cell. The data obtained are shown in Table 4. Incubation with three concentrations of 7β-hydroxy-EPIA for 4hours in the absence of TNF-α caused no significant changes in PGE₂, PGD₂ and 15d-PGJ₂ levels in the culture medium. However, both PGD₂ and 15d-PGJ₂ were markedly increased when cell cultures were incubated with 7β-hydroxy-EPIA for 24h. COX-2 expression was not noticeably changed by the steroid at either 4 or 24 hours, but m-PGES1 mRNA levels showed a significant decrease and increase at 4 hours and 24 hours, respectively.

The presence of TNF-α caused the expected increase in all PG at 24 h. Co-incubation with 7β-hydroxy-EPIA for 4 hours did not further change PG levels at any of the concentrations tested. In contrast, at 24 h, the two lower doses of 7β-hydroxy-EPIA decreased PGD₂ and increased 15d-PGJ₂ levels, when compared with TNF-α alone. Incubation with TNF-α(i.e. inflammatory activation) also led to a marked increase in COX-2 and m-PGES1 mRNA. Increases in COX-2 and m-PGES1 mRNA production by TNF-α were blunted when cell cultures were incubated with 10 and 100nM 7β-hydroxy-EPIA for 24hours.

Altogether, these results clearly indicate that 7β-hydroxy-EPIA surprisingly has significant anti-inflammatory effects.

**Table 2: Effect of three 7α-hydroxy-DHEA doses on PG levels and gene expression in human PBMC cultured with and without TNF-α. * mRNA production relative to controls; ^{a} significantly increased above controls (p<0.05); ^{b} significantly decreased below control levels (p<0.05). ^{c} significantly increased above TNF-α alone (p<0.05); ^{d} significantly decreased below TNF-α alone (p<0.05).**

| Assay | Control | | 7α-hydroxy-DHEA (1 nM) | | 7α-hydroxy-DHEA (10 nM) | | 7α-hydroxy-DHEA (100 nM) | |
|---|---|---|---|---|---|---|---|---|
| Culture duration | 4 h | 24 h | 4 h | 24 h | 4 h | 24 h | 4 h | 24 h |
| PGE2 (ng/mL) | 0.24 ± 0.012 | 0.21 ± 0.015 | 0.22 ± 0.006 | 0.23 ± 0.003 | 0.22 ± 0.006 | 0.30 ± 0.006 | 0.22 ± 0.005 | 0.25 ± 0.012 |
| PGD2 (ng/mL) | 0.15 ± 0.008 | 0.16 ± 0.019 | 0.17 ± 0.004 | 0.13 ± 0.001 | 0.18 ± 0.002 | 0.17 ± 0.003 | 0.17 ± 0.003 | 0.13 ± 0.002 |
| 15d-PGJ2 (ng/mL) | 0.38 ± 0.015 | 0.40 ± 0.048 | 0.46 ± 0.004 | 0.51 ± 0.013^{a} | 0.42 ± 0.010 | 0.59 ± 0.003^{a} | 0.41 ± 0.004 | 0.58 ± 0.024^{a} |
| COX-2 (mRNA*) | 1.00 ± 0.006 | 1.00 ± 0.003 | 0.56 ± 0.072b | 0.82 ± 0.27^{b} | 0.94 ± 0.049 | 1.66 ± 0.408^{a} | 1.10 ± 0.108 | 1.05 ± 0.214 |
| mPGES1 (mRNA*) | 1.00 ± 0.030 | 1.00 ± 0.019 | 0.76 ± 0.053 | 1.77 ± 0.324^{a} | 0.94 ± 0.067 | 3.61 ± 0.662^{a} | 0.71 ± 0.058 | 2.63 ± 0.475³ |
| | | | | | | | | |
| | | | | | | | | |
| | | | | | | | | |

| Assay | TNF-α (10 ng/mL) | | 7α-hydroxy-DHEA (1 nM) + TNF-α (10 ng/mL) | | 7α-hydroxy-DHEA (10 nM) + TNF-α (10 ng/mL) | | 7α-hydroxy-DHEA (100 nM) + TNF-α (10 ng/mL) | |
|---|---|---|---|---|---|---|---|---|
| Culture duration | 4 h | 24 h | 4 h | 24 h | 4 h | 24 h | 4 h | 24 h |
| PGE2 (ng/mL) | 0.43 ± 0.018 ^{a} | 1.26 ± 0.161^{a} | 0.43 ± 0.003 | 1.53 ± 0.048^{a} | 0.45 ± 0.012 | 1.75 ± 0.052^{c} | 0.39 ± 0.009 | 1.40 ± 0.037^{c} |
| PGD2 (ng/mL) | 0.20 ± 0.006 | 0.63 ± 0.029^{a} | 0.20 ± 0.003 | 0.17 ± 0.01 ^{d} | 0.17 ± 0.006 | 0.18 ± 0.008^{d} | 0.18 ± 0.001 | 0.18 ± 0.004^{d} |
| 15d-PGJ2 (ng/mL) | 0.40 ± 0.013 | 1.10 ± 0.082^{a} | 0.37 ± 0.020 | 0.63 ± 0.007^{d} | 0.40 ± 0.004 | 0.71 ± 0.012^{d} | 0.41 ± 0.021 | 0.51 ± 0.012^{d} |
| COX-2 (mRNA*) | 4.49 ± 1.178^{a} | 1.70 ± 0.347^{a} | 4.72 ± 1 .229^{a} | 3.02 ± 0.594 ^{a} | 3.70 ± 1.246 ^{a} | 2.04 ± 0.631^{a} | 3.30 ± 0.655^{a} | 3.13 ± 1.218^{a} |
| mmPGES1(mRNA*) | 1.57 ± 0.148^{a} | 16.1 ± 3.82^{a} | 0.87 ± 0.187 | 21.5 ± 6.66^{a} | 0.76 ± 0.118 | 24.9 ± 10.30^{a} | 0.62 ± 0.066 | 25.4 ± 6.96^{a} |

**Table 3: Effect of three 7β-hydroxy-DHEA doses on PG levels and gene expression in human PBMC cultured with and without TNF-α. * mRNA production relative to controls; ^{a} significantly increased above controls (p<0.05); ^{b} significantly decreased below control levels (p<0.05). ^{c} significantly increased above TNF-α alone (p<0.05); ^{d} significantly decreased below TNF-α alone (p<0.05).**

| Assay | Control | | 7β-hydroxy-DHEA (1 nM) | | 7β-hydroxy-DHEA (10 nM) | | 7β-hydroxy-DHEA (100 nM) | |
|---|---|---|---|---|---|---|---|---|
| Culture duration | 4 h | 24 h | 4 h | 24 h | 4 h | 24 h | 4 h | 24 h |
| PGE2 (ng/mL) | 0.24±0.012 | 0.21 ±0.015 | 0.22±0.003 | 0.22±0.003 | 0.22±0.013 | 0.24±0.002 | 0.22±0.002 | 0.24±0.004 |
| PGD2 (ng/mL) | 0.15±0.008 | 0.16±0.019 | 0.18±0.002 | 0.21 ±0.009^{a} | 0.14±0.001 | 0.24±0.001^{a} | 0.19±0.008 | 0.34±0.025^{a} |
| 15-PGJ2 (ng/mL) | 0.38±0.015 | 0.40±0.048 | 0.45±0.016 | 0.66±0.013^{a} | 0.40±0.014 | 0.62±0.008^{a} | 0.42±0.030 | 0.56±0.020^{a} |
| COX-2 (mRNA*) | 1.00±0.006 | 1.00±0.003 | 1.20±0.179 | 1.76±0.672 | 1.33±0.039 | 1.54±0.630 | 5.09±1.166^{a} | 2.78±0.580^{a} |
| mPGES1 (mRNA*) | 1.00±0.030 | 1.00±0.019 | 0.43±0.057^{b} | 1.94±0.422^{a} | 0.67±0.07^{b} | 1.64±0.338^{a} | 0.82±0.085^{b} | 1.64±0.322^{a} |
| | | | | | | | | |
| | | | | | | | | |
| | | | | | | | | |

| Assay | TNF-α (10 ng/mL) | | 7β-hydroxy-DHEA (1 nM) + TNF-α (10 ng/mL) | | 7β-hydroxy-DHEA (10 nM) + TNF-α (10 ng/mL) | | 7β-hydroxy-DHEA (100 nM) + TNF-α (10 ng/mL) | |
|---|---|---|---|---|---|---|---|---|
| C Culture duration | 4 h | 24 h | 4 h | 24 h | 4 h | 24 h | 4 h | 24 h |
| P PGE2 (ng/mL) | 0.43±0.018^{a} | 1.26±0.161^{a} | 0.34±0.030 | 1.19±0.003^{b} | 0.35±0.002 | 1.2±0.08^{b} | 0.30±0.006 | 1.18±0.010^{b} |
| P PGD2 (ng/mL) | 0.20±0.006 | 0.63±0.029^{a} | 0.16±0.006 | 0.47±0.011^{c} | 0.21±0.007 | 0.42±0.006^{c} | 0.16±0.012 | 0.40±0.041^{c} |
| 1 15d-PGJ2 (ng/mL) | 0.40±0.013 | 1.10±0.082^{a} | 0.44±0.016 | 0.62±0.032^{c} | 0.43±0.003 | 0.54±0.022^{c} | 0.39±0.042 | 0.54±0.029^{c} |
| C COX-2 (mRNA*) | 4.49± 1.178^{a} | 1.70±0.347^{a} | 4.81±1.280^{a} | 1.65 ± 0.331 | 2.67±0.229^{a} | 2.52±0.580 | 3.23±0.242 | 3.07±1.117 |
| mPGES1 (mRNA*) | 1.57 ±0.148^{a} | 16.1 ±3.82^{a} | 0.60±0.050^{d} | 29.2 ±5.19^{a} | 1.01±0.098^{d} | 16.3±3.80^{a} | 0.78±0.055^{d} | 14.0±4.91^{a} |

**Table 4: Effect of three 7β-hydroxy-EpiA doses on PG levels and gene expression in human PBMC cultured with and without TNF-α. •mRNA production relative to controls; ^{a} significantly increased above controls (p<0.05); ^{b}significantly decreased below control levels (p<0.05). ^{c} significantly increased above TNF-α alone (p<0.05); ^{d} significantly decreased below TNF-α alone (p<0.05).**

| Assay | Control | | 7β-hydroxy-EpiA (1 nM) | | 7β-hydroxy-EpiA (10 nM) | | 7β-hydroxy-EpiA (100 nM) | |
|---|---|---|---|---|---|---|---|---|
| Culture duration | 4 h | 24 h | 4 h | 24 h | 4 h | 24 h | 4 h | 24 h |
| PGE2 (ng/mL) | 0.24±0.012 | 0.21±0.015 | 0.26±0.011 | 0.26±0.015 | 0.23±0.022 | 0.23±0.021 | 0.22±0.020 | 0.24±0.022 |
| PGD2(ng/mL) | 0.15±0.008 | 0.16±0.019 | 0.18±0.005 | 0.21±0.012^{a} | 0.19±0.020 | 0.23±0.001^{a} | 0.20±0.013 | 0.24±0.031^{a} |
| 15d-PGJ2 (ng/mL) | 0.38±0.015 | 0.40±0.048 | 0.39±0.080 | 0.85±0.014^{a} | 0.39±0.010 | 0.91±0.032^{a} | 0.37±0.018 | 1.70±0.016^{a} |
| COX-2 (mRNA*) | 1.00±0.006 | 1.00±0.003 | 0.56±0.072^{b} | 0.82±0.27^{b} | 0.94±0.048 | 1.66±0.408 | 1.10±0.108 | 1.05±0.214 |
| m-PGES1 (mRNA*) | 1.00±0.030 | 1.00±0.131 | 0.76±0.053^{b} | 1.77±0.324^{a} | 0.94±0.067 | 3.61±0.662^{a} | 0.71±0.058^{b} | 2.63±0.475^{a} |
| | | | | | | | | |
| | | | | | | | | |
| | | | | | | | | |

| Assay | TNF-α (10 ng/mL) | | 7β-hydroxy-EpiA (1 nM) + TNF-α (10 ng/mL) | | 7β-hydroxy-EpiA (10 nM) + TNF-α (10 ng/mL) | | 7β-hydroxy-EpiA (100 nM) + TNF-α (10 ng/mL) | |
|---|---|---|---|---|---|---|---|---|
| Culture duration | 4 h | 24 h | 4 h | 24 h | 4 h | 24 h | 4 h | 24 h |
| PGE2 (ng/mL) | 0.43±0.018^{a} | 1.26±0.161^{a} | 0.50±0.012 | 0.70±0.001^{b} | 0.49±0.021 | 0.80±0.012^{b} | 0.42±0.020 | 0.71±0.012^{b} |
| PGD2 (ng/mL) | 0.20±0.006 | 0.63±0.029^{a} | 0.22±0.082 | 0.47±0.013^{d} | 0.22±0.010 | 0.42±0.009^{d} | 0.23±0.007 | 0.40±0.030^{d} |
| 15d-PGJ2 (ng/mL) | 0.40±0.013 | 1.10±0.082^{a} | 0.37±0.029 | 2.18 ±0.183^{c} | 0.40±0.007 | 2.77±0.021^{c} | 0.37±0.016 | 0.85±0.019 |
| COX-2 (mRNA*) | 4.49±1.178^{a} | 1.70±0.347^{a} | 7.39±0.282^{c} | 2.74±0.555^{c} | 2.75±0.147^{d} | 0.94±0.194^{d} | 5.76±0.660 | 0.67±0.132^{d} |
| m-PGES1 (mRNA*) | 1.57±0.148^{a} | 16.1±3.82^{a} | 1.11±0.235 | 21.6 ±4.28 | 1.15±0.183 | 6.29±1.131^{d} | 1.29±0.323 | 6.79±2.898^{d} |

### EXAMPLE 4

### Effect of 7β-OH-EPIA on an experimental model of colitis

Administration to rats of dextran sodium sulphate (DDS) in the drinking water for 6 consecutive days causes colonic inflammation (colitis) characterized by colon length reduction, increased MPO activity, mucus depletion in goblet cells, and increased expression of COX-2 and mPGES-1 synthase and production of prostaglandin E2 (PGE2). Administration of DSS also increases oxidative stress markers such as protein carbonyl (Prot CO) and Tbars in the gut. We now provide evidence that treatment with 7β-hydroxy-EPIA once a day for 7days before administration of DSS can prevent the development of DSS-induced colitis. Administration of 0.01mg/kg 7β-hydroxy-EPIA completely prevented colitis damage and tissue inflammation, and this effect of 7β-hydroxy-EPIA was associated with a marked reduction in oxidative stress markers and PGE₂ production, associated with an early but transient increase in COX-2 expression and a sustained increase in the production of the anti-inflammatory prostaglandin 15d-PGJ₂. These results show that 7β-hydroxy-EPIA has marked anti-inflammatory effects at very low dose levels in this accepted experimental model of Inflammatory Bowel Disease (IBD).

### Experimental Procedures

### Animals

All experimental protocols and procedures were in agreement with the directive 86/609/CEE of the European Community, for the use of laboratory animals. Male Wistar rats (180-200 g), purchased from Charles River (L'Arbresle, France) were fed with rodent laboratory chow and were given water *ad libitum.*

### Drug treatment and colitis induction

After a 7-day adaptation period, animals were divided into two control groups (sham-control and 7β-hydroxy-EPIA-treated control group and two colitis groups (colitis and 7β-hydroxy-EPIA-treated colitis). 7β-Hydroxy-EPIA (0.01, 0.1, and 1mg/kg body weight dissolved in DMSO) or DMSO alone (vehicle) was administered *i.p.* once a day for 7 days, from day 0 to day 7. Colitis was induced from day 7 to 14 by addition of DSS (molecular weight 36-50 kDa; MP Biomedicals, France) to drinking water. The two control groups received tap water only.

### Macroscopic assessment of colon damage

At day 9, 11, 13, 14, body weight, colon length, stool consistency, were recorded and fresh rectal bleeding evaluated by ocular inspection. The severity of colonic damage was blindly scored as described by Mabley et al., 2001, Inflamm. Res.; 50: 561-569) (0: well-formed pellets and no colon damage; 1: colon with a small amount of blood present mixed with faeces; 2: colon with large amount of blood present with faeces; 3: colon filled with blood and no faeces).

### Histological examination

A portion of the proximal colon (1cm) was fixed in 4% formaldehyde (Labonord, Templemars, France) and embedded in paraffin. Tissue sections (5 µm) were prepared, thereafter cleared, hydrated and stained either with hematoxylin/eosin or with Alcian blue according to standard protocols for histological evaluation of colonic damage and mucus goblet cells content, respectively.

### Preparation of tissue homogenates

The colon was opened along the mesenteric border, and the epithelial cells were scrapped away with the blunt edge of a glass slide then weighed, washed and centrifuged. The pellet was homogenized in 9 volumes TKE buffer (10 mM Tris-HCl; 150 mM KCl; 1 mM EDTA; 0.25 mM PMSF, pH 7.4) and then frozen at -80°C until further use. The protein content of homogenates were measured according to Lowry et al. (JBiol Chem 1951;193: 265-74).

### MPO activity

MPO activity was assessed in homogenates using the o-dianisidine method of Krawisz et al. (Gastroenterology 1984;87(6):1344-50) with the modifications of Pelissier et al. (Steroids 2006;71(3):240-8). MPO activity was expressed as the amount of enzyme necessary to produce a per minute change in absorbance at 460 nm using the extinction coefficient for oxidized o-dianisidine (1.13×10⁴ mol⁻¹.cm⁻¹).

### Biochemical determination of oxidative stress

Lipid peroxidation (Tbars) was measured by a modification by Albrecht et al., (1992, Toxicol. Lett; 63: 91-96) of the method described by Ohkawa et al. (Anal Biochem 1979; 95:315-58). Absorbance of the red 1:2 adduct malondialdehyde-thiobarbituric acid (Sigma-Aldrich, St Quentin-Fallavier, France) was measured at 532nm (extinction coefficient used: 0.156 µmol⁻¹ cm⁻¹). The carbonyl contents in oxidized proteins (Prot CO) were assessed by the method of Levine et al. (Methods Enzymol 1990;186:464-78). The non-protein sulfhydryl group content (mostly GSH) was taken as the anti-oxidant defense marker in homogenates and determined by the method of Sedlak and Lindsay (Anal Biochem 1968;25(1):192-205).

### Prostaglandin immunoassays

Commercially available EIA kits were used for the determination of PGE₂ levels (Oxford Biomedical Research, UK) and 15d-PGJ₂ levels (Assay designs, Euromedex, France) in colonic supernatants obtained from fresh homogenates. Measurement of PGD₂ levels was obtained using the prostaglandin D₂-MOX EIA kit (Cayman Chemical, Euromedex). In this case, and prior to assay, the fresh samples were immediately treated with the MOX-HCl reagent which converted PGD₂ into PGD₂-MOX, thus preventing any further chemical degradation.

### Real Time reverse transcriptase PCR

Total RNA from fresh colonic samples (300mg) was extracted using Trizol Reagent (Invitrogen, Cergy-Pontoise, France). Poly(A) RNA was purified from total RNA with MicroPoly(A)Purist kit (Ambion, Huntingdon, UK). cDNA was synthesized using the Superscript first strand synthesis system kit (Invitrogen). Real-time PCR was performed using mixtures (25 µL) containing 2.5x RealMaster Mix/20x SYBR solution (11.25 µL) (Eppendorf, Le Pecq, France) and 300 nM forward and reverse primers. Reactions were run on a RealPlex ep gradient S mastercycler (Eppendorf). The cycling conditions were at 95°C for 10 minutes and 40 cycles at 95°C, 55°C and 68°C for 15 seconds, 30 seconds and 30 seconds, respectively. Each assay included a standard curve of four serial dilution points of control cDNA. Oligonucleotide primers were synthesized by Genecust/Distribio (Evry, France). The specificity of the amplified product was monitored by examination of the product melting curve and confirmed by analysis on agarose gel electrophoresis. The levels of mRNA were expressed relative to that of the DMSO control, after normalization to HPRT1.

### Statistical analysis

All assays were carried out in triplicate for each animal and results are plotted as mean±S.E.M. One-way analysis of variance was performed followed by Duncan's multiple range tests in order to compare the differences between groups. Differences were considered statistically significant when p<0.05.

### RESULTS

### Time course study of colitis induction

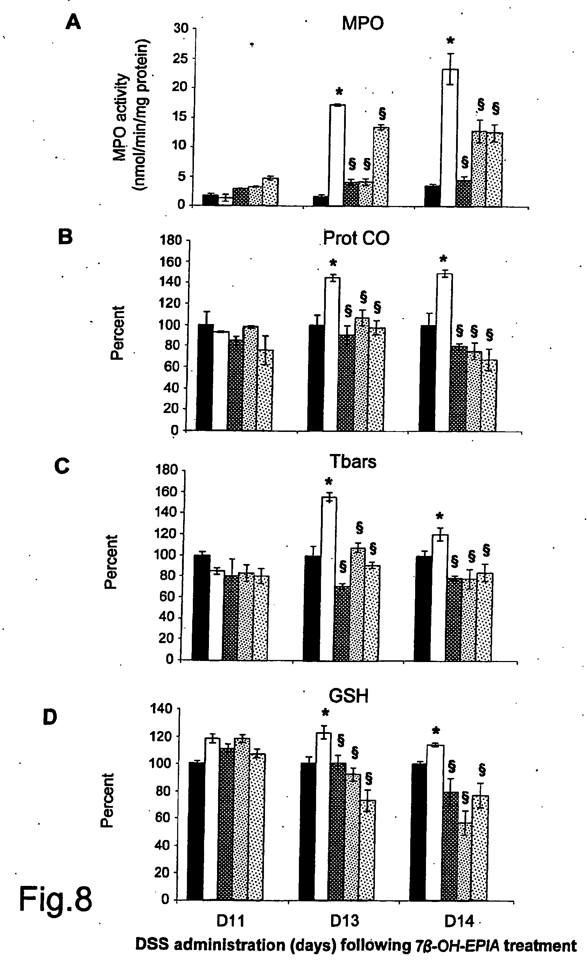

Treatment of rats with 5% DSS in the drinking water for 7 days (from D7-D14) resulted in clinical and histological signs of colitis without mortality. Typically, all the rats exhibited severe diarrhea 5 days after colitis induction (day 12) and rectal bleeding occurred the next day. A decrease in body weight, epididymal adipose tissue mass and liver weight was recorded at day 11 in all DSS-treated rats when compared with sham-controls (-5%, -17%, -8%; p<0.05, respectively). These decreases were also observed at day 14 (Table 5). No change in spleen weight was observed. When compared with the sham-control group, DSS-treated rats also demonstrated a significant shortening of the colon from day 11 (-14%, p<0.05) to day 14 (-26%; p<0.05), see Table 5, associated with a thickened colonic tissue and faecal stool loss. MPO activity in the colonic mucosa was measured as an index of neutrophil infiltration. At day 13 and 14, a 9-fold and 7-fold increase of mucosal MPO activity was observed in the colitis group when compared with sham-controls (p<0.05) (Fig. 8A). No modification of MPO activity was observed prior to day 13. These results are consistent with histological analysis. Indeed, major hallmarks of colonic inflammation, namely cryptic distortion, neutrophil infiltration into the mucosal tissue and loss of goblet cells which contained less mucins were apparent in the colitis group at day 13 and were more pronounced at day 14 when compared with sham-control group (data not shown).

As shown in Fig. 8, the two markers for oxidative stress, namely Prot CO, Tbars and the anti-oxidant defense parameter GSH levels, were significantly increased above control levels at day 13 and 14 in the colonic mucosa of animals with colitis.

### Protective effect of 7β-hydroxy-EPIA on colitis

The two low doses of 7β-hydroxy-EPIA (0.01, 0.1 mg/kg) prevented the DSS-induced colonic damages as indicated by the suppression of diarrhea and rectal bleeding at day 14. Intra peritoneal injection of 0.01 mg/kg 7β-hydroxy-EPIA once daily for 7days before the administration of DSS restored body weight to control levels without altering the weight of the epididymis (Table 5). Between day 9 and day 14, colon length reduction was less pronounced in the groups treated by the two lower doses of the steroid (0.01, 0.1 mg/kg), than in the colitis group (data not shown for day 9, Table 5). 7β-Hydroxy-EPIA prevented mucus depletion in goblet cells at day 14 and histological changes such as the abnormality of crypts and neutrophil infiltration (data not shown) and significantly decreased MPO activity at all dose levels, 0.01, 0.1 and 1 mg/kg (Fig. 8A). The oxidative stress parameters (Prot CO, Tbars) and GSH levels were similar at days 9 (not shown) and 11 in all groups, namely sham-control, colitis and 7β-hydroxy-EPIA- treated (0.01, 0.1 and 1 mg/kg) colitis group (Fig. 8B, C and D). All markers of oxidative stress and antioxidant defense marker remained unchanged in animals that had been treated with 7β-hydroxy-EPIA (from DO-Day7) while these parameters were significantly increased in the colitis group (p<0.05). These results show that 7β-hydroxy-EPIA has marked anti-inflammatory effects at very low dose levels in this experimental model of Inflammatory Bowel Disease (IBD).

**Table 5: Final body and organ weight, length of colon, macroscopic assessment of colon damage score in rats with DSS-induced colitis (DSS (5%) in tap water for 7 days (Day7-Day14). The data were obtained at the end of the experiment (day 14) and are expressed as mean±SEM.Values not sharing the same superscript letter (a-c) within a line, are significantly different (p<0.05). Scoring of colon damage was according to Mableet al. (2001, Inflamm. Res.,50 ,561-569 )y(0: well formed pellets no colon damage; 1: colon with small amount of blood present with faeces; 2: colon with large amount of blood present with faeces; 3: colon filled with blood no faeces). * mg/kg body weight.**

| **Group** | **Control** | **Colitis** | **7β-Hydroxy-EPIA pre-treated colitis group** | | |
|---|---|---|---|---|---|
| | 0 | 0 | 0.01* | 0.1* | 1* |
| **Number of rats (n)** | 15 | 23 | 3 | 12 | 12 |
| Body weight (g) | 304.6±6^{a} | 279.6±6.5^{b} | 297.5±9.5^{a} | 272.1±6^{b} | 267.3±7.7^{b} |
| Epididymis weight (g) | 2.5±0.08^{a} | 2.1±0.1^{b} | 2.5±0.03^{a} | 1.9±0.14^{b} | 1.9±0.1^{b} |
| Liver weight (g) | 12.7±0.4^{a} | 11.1±0.3^{b} | 12.1±0.3^{a} | 12.3±0.6^{a} | 11.9 ±0.4^{a} |
| Spleen weight (g) | 0.9±0.05^{a} | 0.9±0.08^{a} | 0.8±0.02^{a} | 1.03±0.2^{a} | 0.82±0.06^{a} |
| Colon length (cm) | 18.4±0.6^{a} | 13.7±0.4^{b} | 16±0.01^{c} | 15.6±0.3^{c} | 14.2±0.6^{b} |

### Prostaglandin (PG) production in colonic tissue: effects of β-hydroxy-EPIA

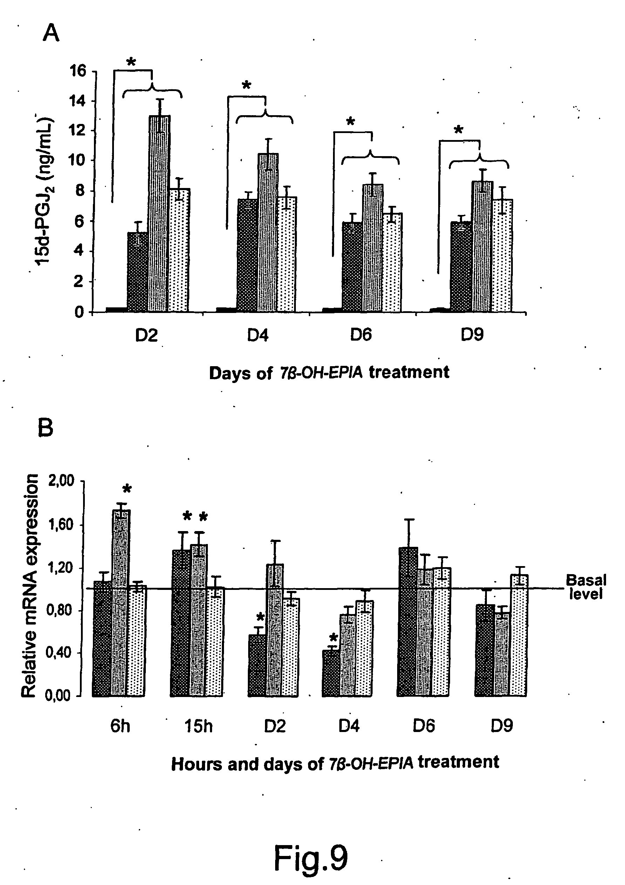

7β-Hydroxy-EPIA treatments did not alter PGE₂ and PGD₂ colonic tissue levels in control rats without colitis (data not shown). In contrast, administration of 7β-hydroxy-EPIA from day 0 to day 7 led to a significant increase above basal levels of 15d-PGJ₂ levels from day 2 to day 14. Administration of 0.1 mg/kg 7β-hydroxy-EPIA resulted in a 51-fold increase at day 2 that decreased progressively from day 4 to day 14 (increments ranging from of 44-fold to 5-fold) (Fig. 9A, Fig. 10C).

Administration of the inflammatory agent DSS from day7 to day14 resulted in marked increases in the production of the pro-inflammatory prostaglandin PGE₂ at day 14, whereas levels of the anti-inflammatory prostaglandin 15d-PGJ₂ were sharply reduced (Fig. 10). Treatment with 0.01mg/kg 7β-Hydroxy-EPIA from D0 to D7 completely prevented DSS-induced production of colonic PGE₂ at day while dramatically increasing the level of the 15d-PGJ2 (Fig. 10).

### COX-2 and PG synthase expression in sham and colitis groups: effect of 7β-hydroxy-EPIA pre-treatment

Because significant modifications in prostaglandin levels were observed with DSS administration and 7β-hydroxy-EPIA treatments, we tested whether the expression of COX-2, mPGES-1 and H-PGDS was altered by steroid pre-treatment by quantifying specific mRNA by real-time RT-PCR. Transcription of these genes was examined and related to that of the HPRT1 house-keeping gene. In control rats without colitis, administration of 0.1 mg/kg 7β-hydroxy-EPIA induced a significant 1.5 fold increase in COX-2 mRNA levels at 15h followed by a significant decrease at day 2 and day 4 thereafter returning to basal values (Fig. 9B). mPGES-1 mRNA expression increased transiently between 6h and 15h returning towards basal levels at day 2. H-PGDS mRNA synthesis remained unchanged during the course of the experiment.

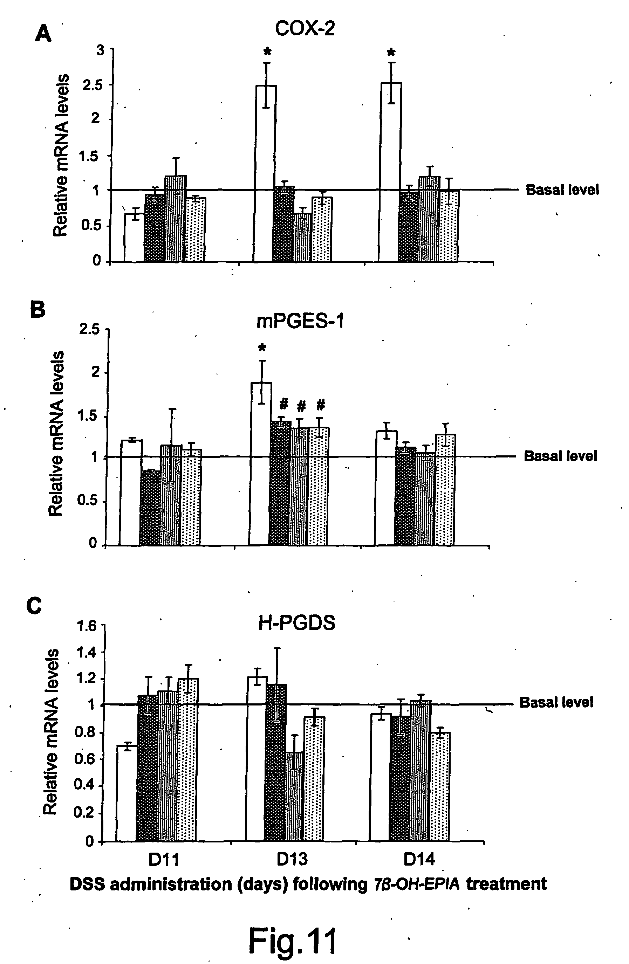

After colitis induction by DSS, a 2.5 fold increase in COX-2 mRNA expression was observed at day 13 and 14 (Fig. 11A) while mPGES-1 mRNA was significantly augmented on day 13 only (Fig. 11B). H-PGDS was not altered by DSS administration.

Pre-treatment with 7β-hydroxy-EPIA (from day0 to day7) suppressed these increase in both COX-2 and mPGES-1 mRNA synthesis by DSS (Fig. 11).

Altogether, these findings indicate that the anti-inflammatory effects of 7β-hydroxy-EPIA are mediated through a concomitant decrease in PGE₂ and an increase in 15d-PGJ₂ production. The long-lasting effect of 7β-hydroxy-EPIA on 15d-PGJ₂ production seen in this study, suggests that 7β-hydroxy-EPIA causes sustained changes in the expression of genes involved in inflammation and its resolution.

In the drawings, Figure 8 shows the effects of 7β-hydroxy-EPIA treatment on (A) myeloperoxidase (MPO) and oxidative stress markers namely (B) Prot CO, (C) Tbars and (D) the antioxidant marker GSH in colonic tissue. Biomarker levels show that DSS-treated rats differed significantly from controls at day 13 and 14 (p<0.05). 7β-hydroxy-EPIA significantly reduced MPO activity at day 13 and 14 compared to colitis group (p<0.05) and restored oxidative stress parameters (B, C and D) to control levels at day 13 and 14 at all dose levels. Values are mean±SEM (A) and percent to sham group (B, C and D) with 3-23 rats per group. In Figures 8A to 8D, the black bars = Sham-control; the white bars = DSS-colitis group; the dark grey bars = DSS + 0.01mg 7β-hydroxy-EPIA; the medium grey bars = DSS + 0.1 mg/kg 7β-hydroxy-EPIA; and the light grey bars = DSS + 1.0 mg/kg 7β-hydroxy-EPIA.

DSS-colitis versus sham-control group (p<0.05); § 7β-hydroxy-EPIA -treated groups versus DSS-colitis group (p<0.05).

Figure 9 shows the colonic 15d-PGJ2 level (A) and quantification of relative mRNA expression of COX-2, mPGES-1 and H-PGDS (B) at various times during 7β-hydroxy-EPIA treatment. Values are mean±SEM (n=3 to 15) (A) and mRNA are expressed relative to that of the sham-control, after normalization to HPRT1 (B). In Figure 9A, the black bar = Sham-control group; the dark grey bar = 0.01mg/kg 7β-hydroxy-EPIA; the medium grey bar = 0.1 mg/kg 7β-hydroxy-EPIA; and the light grey bar = 1.0 mg/kg 7β-hydroxy-EPIA. In Figure 9B, the dark grey bar with white dots = COX-2; the light grey bar with black dots = mPGES-1; and the white bar with black dots = H-PGDS.

Figure 10 shows the effect of 7β-hydroxy-EPIA on colonic synthesis of prostaglandin E2, D2 and 15d-PGJ2 during DSS administration. Administration of DSS increased PGE2 (A), D2 (B) and 15d-PGJ2 (C) synthesis significantly (p<0.05) at day 13 and 14 (p<0.05). Treatment with 7 β -hydroxy-EPIA for 7 days before administration of DSS reduced PGE2 synthesis at day 13 and 14 (A) while increasing 15d-PGJ2 production significantly at all dose levels (C). Data are expressed as mean± SEM with n=3 to 23. In Figures 10A to 10C, the black bar = Sham-control; the white bar = DSS-colitis group; the dark grey bar with white dots = DSS + 0.01mg/kg 7β-hydroxy-EPIA; the light grey bar with black dots = DSS + 0.1 mg/kg 7β-hydroxy-EPIA; and the white bar with black dots = DSS + 1.0 mg/kg 7β-hydroxy-EPIA.

Figure 5 shows the colonic expression of COX-2, mPGES-1 and H-PGDS mRNA during colitis induction. Colitis led to a significantly increased COX-2 mRNA synthesis at day 13 and 14 (A) while mPGES-1 augmented only at day 13 (B). A return towards basal levels of gene expression was observed with all doses of 7 β-hydroxy-EPIA. In Figures 10A to 10C, the white bar = DSS-colitis group; the dark grey bar = DSS + 0.01mg/kg 7β-hydroxy-EPIA; the medium grey bar = DSS + 0.1 mg/kg 7β-hydroxy-EPIA; and the light grey bar = DSS + 1.0 mg/kg 7β-hydroxy-EPIA.

### EXAMPLE 5

### 1. The effect of 7 β-OH-EPIA in collagen induced arthritis

This study tested the effectiveness of 7β-OH-EPIA at controlling the inflammatory and pathological changes associated with a model of collagen-induced arthritis. The murine model demonstrates clinical disease reminiscent of human arthritis.

Male DBA/1 mice (10-12 weeks old) were given 100µg chicken type II collagen emulsified into complete Freund's adjuvant (CII/CFA) on day 0 by injection at the base of the tail. Treatments were given daily from day 20 to 50 by subcutaneous injection (Groups 2, 3 and 4), and from day 0 to 30 by subcutaneous injection (Group 5) as described below:

**Experimental groups (n=10/group)**

| | |
|---|---|
| Group 1: | day 0 CII/CFA, untreated-sham treated day 20 to 50 |
| Group 2: | day 0 CII/CFA, treatment 7β-hydroxy-EPIA 1µg/kg, day 20 to 50 |
| Group 3: | day 0 CII/CFA, treatment 7β-hydroxy-EPIA 10µg/kg, day 20 to 50 |
| Group 4: | day 0 CII/CFA, treatment 7β-hydroxy-EPIA 100µg/kg, day 20 to 50 |
| Group 5: | day 0 CII/CFA, treatment 7β-hydroxy-EPIA 10µg/kg, day 0 to 30 |
| Group 6: | day 0 CII/CFA, untreated-sham treated day 0-30 |

At termination, hind legs were removed and knee homogenates were collected for prostaglandin measurement (groups 1 & 4 only) and paws were fixed for pathology examination by standard methods. Each paw was hemi-sectioned longitudinally, dissected and decalcified to permit sectioning. The decalcified samples were routinely processed, sectioned and one stained section was prepared for examination. This included both halves of each specimen. Each paw was scored according to standard scoring system. Samples were scored in blinded fashion, without knowledge of the experimental protocol or identity of groups.

Clinical joint swelling was scored twice weekly from day 21 to 50. On each occasion, each of the four limbs was given a score according to the following scoring system (combined clinical disease score):
0 normal
1 slight swelling of whole joint or individual digit inflammation
2 intermediate swelling of whole joint with redness and/or inflammation in more than one digit
3 severe joint inflammation and redness spreading to multiple digits
4 severe joint inflammation and redness spreading to multiple digits, overt signs of bone remodelling.
The results are shown in Table 6. It can be seen that treating with 7β-OH-EPIA led to a marked reduction of joint swelling as measured by the above clinical disease scoring method, when compared to control groups (the sham control groups 1 and 6 have been combined). This finding was confirmed by pathological evidence of reduced swelling in the paws (Table 7). From Table 7 it can be seen that the lowest pathology score where 9 out of 10 animals were completely clear of histopathological change in the paw joints, where 7β-hydroxy EPIA treatment commenced at the same time (day 0) as when the collagen was given to induce the arthritis and treatment continued until day 30. Thus, early treatment with 7β-hydroxy EPIA of diseases such as rheumatoid arthritis can have beneficial effects.

### 2. Measurement of prostaglandins in the knee homogenates

Levels of 15d-PGJ2 and PGE2 in knee homogenates as prepared above were measured using standard assay techniques. It can be seen from Table 8 that the levels of PGE2 were lower in the group treated with 0.1mg/ 7β-OH-EPIA when compared to the control untreated group. The group treated with 0.1mg/kg 7β-OH-EPIA had higher levels of 15d-PGJ2 when compared to untreated control.

### Conclusion:

There was a clear anti-inflammatory effect of7β-hydroxy-EPIA. Treatment at the time of induction appeared to have the greatest effect. This is in keeping with other test compounds including steroids in this model and reflects the relative ease of preventing joint damage as opposed to reversing existing changes. Having said this, the levels of disease in those animals treated with the higher doses of 7β-hydroxy-EPIA were similar when treatment was delayed until day 20. This level of disease protection is good for a therapeutic regimen. The lowest dose of 7β-hydroxy-EPIA appeared to have less effect on disease levels, although statistical analysis would be required to investigate whether this was clear dose effect.

Analysis of Prostaglandin E2 and 15d-J2 levels showed a clear effect of treatment. Levels of PGE2 were lower in treated mice as compared to untreated controls. In contrast, levels of 15d-PGJ2 were increased following treatment.

Taken together the clinical disease observations and the histopathology provide strong evidence for an effect anti-inflammatory of 7β-hydroxy-EPIA in this model of arthritis. Treatment prior to disease onset was most effective, as is almost invariably the case in this model. However, the finding that 7β-hydroxy-EPIA can suppress disease progression when given around the time of onset marks this compound out from many competitors and is very encouraging.

**Table 6**

| Effect of 7β-hydroxy-EPIA on Histopathology in CIA Rats | | |
|---|---|---|
| Treatment from Day 20-50 | | |
| Group | n | Histopathology Score (both paws on Day 50) |
| Sham - untreated | 10 | 1.35 |
| 7β-hydroxy-EPIA 1.0 µg/kg | 10 | 0.60 |
| 7β-hydroxy-EPIA 10.0 µg/kg | 9 | 0.44 |
| 7β-hydroxy-EPIA 100.0 µg/kg | 10 | 0.85 |

| | | |
|---|---|---|
| From these results it may be concluded that 7β-hydroxy-EPIA at doses as low as 1.0 µg/kg is able to reduce joint damage. | | |

**Table 7**

| Effect of 7β-hydroxy-EPIA on Histopathology in CIA Rats | | |
|---|---|---|
| Treatment from Day 0-30 | | |
| Group | n | Histopathology Score (both paws on Day 50) |
| Sham - untreated | 9 | 1.22 |
| 7β-hydroxy-EPIA 10.0 µg/kg | 10 | 0.2 * |

| | | |
|---|---|---|
| * 9 out of 10 animals were completely clear of histopathological changes. | | |

From these results, it may be concluded that 7β-hydroxy-EPIA at 10.0 µg/kg almost completely prevents the development of disease, i.e. joint damage.

**Table 8**

| Effect of 7β-hydroxy-EPIA (0.1 µg/kg day, D 20-50) joint swelling and prostaglandin content in CIA Rats on Day 50 | | |
|---|---|---|
| | Untreated | Treated |
| Clinical Score | 7.50 ± 2.51 | 3.10 ± 2.96 |
| PGE₂ (pg/ml) | 177.09 ± 70.08 | 95.61 ± 34.65 |
| 15d-PGJ₂ (pg/ml) | 19.12 ± 6.43 | 78.96 ± 22.18 |
| PGE₂/15d-PGJ₂ | 9.26 | 1.21 |

From these results, it may be concluded that 7β-hydroxy-EPIA reduces the severity of the clinical symptoms of collagen-induced arthritis and alters tissue prostaglandin production in favour of resolution of inflammation, cell protection and tissue repair.

### EXAMPLE 6

### Effect of 7β-OH EPIA on a cisplatin-induced peripheral neuropathy model

Cisplatin is an antimitotic compound used for the treatment of cancers. However, its use is limited by several adverse-effects, among which the peripheral neuropathies are particularly distressing. Cisplatin-induced neuropathies are predominantly sensitive neuropathies. Patients suffer from sensitivity losses in distal extremities followed by sensory ataxia. Histological studies showed axonal degeneration. In cell cultures, treatment of sensitive neurones by cisplatin induced a decrease of neurite network density followed by cell body degeneration. Nerve Growth Factor (NGF), a specific growth factor for sensory neurones, has protective effects on neurones against this intoxication. Sensory neurones intoxication in culture by cisplatin is thus an adequate model for the study of neuroprotective effects of compounds in peripheral neuropathies.

Neuroprotective effects of 7β-OH EPIA were evaluated on rat sensory neurones in a model of peripheral neuropathy.

Primary cultures of dissociated dorsal root ganglia sensory neurones were incubated with 3µ/ml cisplatin, with and without 7β-OH EPIA for 48 hours and 72 hours and the following parameters were evaluated:
- neurone cell bodies stained with an anti MAP 2 antibody (microtubule associated protein 2)
- neurite network density stained with an anti β-Tubulin antibody

### Cultures of neurones

Rat sensory neurones were prepared according to the method described by Hall et al 1997 [Hall et al. (J. Neurosci. 1997 Apr 15; 17(8); 2775-84]. Briefly, a female rat (15 days gestation) was killed by cervical dislocation (Rats Wistar; Janvier, Le Genest-St-Isle, France) and the foetuses removed from the uterus. Their spinal cords with the dorsal root ganglia (DRG) were removed and placed in ice-cold medium of Leibovitz (L15, Fisher 11415-049) containing Penicillin 50 UI/ml - Streptomycin 50 µg/ml (PS, 1%) and bovine serum albuminutes (BSA 1%, Sigma A6003). The DRG were recovered and dissociated by trypsinisation for 20 minutes at 37 °C (trypsin EDTA 10X, 10 %, Fisher 15400054) diluted in PBS without calcium and magnesium (Fisher 2007-03). The reaction was stopped by addition of Dulbecco modified Eagle medium (DMEM, Fisher 21969-035) containing DNase I grade II (0.1 mg/ml Roche diagnostic 104159) and foetal bovine serum (FBS 10%, Fisher 10270-098). The cell suspension was triturated with a 10 ml pipette and centrifuged at 350 x g for 10 minutes at room temperature. The pellet of dissociated cells was then resuspended in defined culture medium.

Viable cells were counted in a Neubauer cytometer using the trypan blue exclusion test (Sigma) and seeded at a density of 30 000 cells/well in 96 well-plates (Nunc). Wells were pre-coated with poly-L-lysine (10 µg/ml, Sigma P2636) in ultra pure sterile water (Merck Eurolab 60759.01).

Cells were allowed to adhere for 2h and maintained in a humidified incubator at 37 °C in 5% CO₂/95% air atmosphere.

### Incubation of neuronal cultures with 7β-OH EPIA

After 5 days of culture, the culture medium was changed into a defined culture medium following the different conditions described below:
- Vehicle (DMSO 0.1 %)
- Vehicle (DMSO 0.1%) + cisplatin (3µg/ml, Sigma ref: p4394)
- Test compound 7β-OH EPIA (1 nM, 10 nM and 100nM) + cisplatin (3µg/ml)
- Reference compound NGF (10 ng/ml) + cisplatin (3µg/ml)

Six wells per condition were carried out to assess neuronal survival. After 48 hours and 72 hours of incubation, the neuronal cells were fixed for 5 minutes in an ethanol/acetic acid solution (95%/5%) at -20°C and rinsed 3 times in PBS.

For the control of the neurotrophic effect of compounds, NGF (10 ng/ml), and 7β-OH EPIA (1 nM, 10 nM and 100 nM) were incubated during 48 hours and 72 hours. At the end of the incubation, the cells were fixed for 5 minutes in an ethanol/acetic acid solution (95%/5%) at -20°C and rinsed 3 times in PBS.

### Analysis of number of cell bodies and neurite network per field

Cell bodies of sensory neurones were labelled by monoclonal anti MAP-2 antibody (Sigma M4403) and the neurite of sensory neurones were labelled by monoclonal β-Tubulin antibody (Sigma T8660). These antibodies were diluted at 1:400 in incubation solution (PBS with 5% of FCS and 0.1% of saponin, Sigma S-7900). These antibodies specifically label neurone cell bodies and neuritis, respectively.

After 2 hours of incubation, the cells were washed in PBS and incubated with Alexa Fluor 488 goat anti mouse IgG (Molecular Probes A11001) diluted at 1:300 in incubation solution to reveal the MAP-2 and the β -Tubulin antibodies. Cell nuclei were stained with a fluorescent marker (Hoechst staining solution, SIGMA H6024, 1 µg/ml in incubation solution during 1 hour).

For each condition, 2 pictures per well (12 pictures per condition) were taken using In Cell Analyzer 1000 (Amersham Biosciences) controlled by the computer software In Cell Analyzer 1000 3.2. For the MAP-2 labelling, the magnification was x10, for the β-Tubulin labelling, the magnification was x20. For each labelling, all the images were taken in the same conditions.

Analysis of the number of cell bodies labelled with anti-MAP-2 antibodies, and total length of neurites labelled with anti β -Tubulin antibodies, were carried out using In Cell Analyzer 1000 3.2.Workstation software. The results were expressed in percentage compared to the vehicle. The comparisons of each group were carried out using the unpaired T test.

### RESULTS

### Protection by 7β-OH EPIA against cisplatin-induced loss of neurite density

### Incubation with 3µg/ml cisplatin for 48 hours

Neurite density expressed a mean of 5459 µm of total neurite length per field after incubation of sensory neurones with medium ("vehicle"), i.e. without cisplatin for 48hours. Incubation with cisplatin for 48hours reduced the total neurite length per field to approx. 4585 µm. This reduction in neurite network density by cisplatin was statistically significant (-16 %, p<0.001) when compared to vehicle.

Incubation with 10 ng/ml NGF for 48hours prevented cisplatin-induced loss of neurites and caused a significant increase in neurite length when compared with cultures incubated with medium only.

Incubation with 1nM, and 10nM) 7β-OH EPIA protected sensory neurones against cisplatin-induced neurite loss at 48hours. This effect was statistically significant. Total neurite length was 5378µm and 5549 µm after 48 hours of incubation with 1nM and 10nM 7β-OH EPIA respectively, which represents a reduction of cisplatin-induced toxicity of respectively 90.7% and 101%.

### Incubation with 3µg/ml cisplatin for 72 hours

Sensory neurones in medium "vehicle" without cisplatin expressed a mean of 5320 µm of total neurite length per field. Incubation with cisplatin for 72hours reduced the total neurite length per field to approx. 4046 µm. This reduction in neurite network density by cisplatin was statistically significant (-24 %, p<0.001) when compared to vehicle.

Incubation with 10 ng/ml NGF for 72 hours prevented cisplatin-induced loss of neurites and caused a significant increase in neurite length when compared with cultures incubated with medium only.

Incubation with 1nM 7β-OH EPIA protected sensory neurones against cisplatin-induced neurite loss at 72 hours. The effect was statistically significant. Total neurite length was 4948µm after 72hours of incubation with 1nM 7β-OH EPIA, which represents a reduction of cisplatin-induced toxicity of 70.8%.

**Table 9**

| Effects of the vehicle (0.1% DMSO), NGF (10 ng/ml) and 7β-OH EPIA (1 nM, 10 nM and 100 nM) on neurite length of sensory neurones after 48 hours incubation in presence of cisplatin (3 µg/ml). | | | | | | |
|---|---|---|---|---|---|---|
| Treatment | Conc. | Mean neurite length (µm) | SEM | n | % Control without cisplatin | p (compared to vehicle + cisplatin) |
| Vehicle (0.1% DMSO) | - | 5459.72 | 184 | 12 | 100 | p<0.001 |
| Vehicle (0.1% DMSO) intoxication with 3 µg/ml of cisplatin | - | 4585.52 | 133 | 12 | 84 | - |
| NGF intoxication with 3 µg/ml of cisplatin | 10 ng/ml | 6362.44 | 297 | 12 | 117 | p<0.001 |
| 7β-OH EPIA intoxication with 3 µg/ml of cisplatin | 100 nM | 4442.51 | 125 | 12 | 81 | p>0.05 |
| | 10 nM | 5549.41 | 146 | 12 | 102 | p<0.001 |
| | 1 nM | 5377.89 | 106 | 12 | 99 | P<0.001 |

**Table 10**

| Effects of the vehicle (0.1% DMSO), NGF (10 ng/ml) and 7β-OH EPIA (1 nM, 10 nM and 100 nM) on the neurite length of sensory neurones after 72 hours of incubation in presence of cisplatin (3 µg/ml) | | | | | | |
|---|---|---|---|---|---|---|
| Treatment | Conc. | Mean neurite length (µm) | SEM | n | % Control without cisplatin | p (compared to vehicle + cisplatin) |
| Vehicle (0.1% DMSO) | - | 5320.43 | 156.74 | 12 | 100 | p<0.001 |
| Vehicle (0.1% DMSO) intoxication with 3 µg/ml of cisplatin | - | 4046.14 | 138.52 | 12 | 76 | - |
| NGF intoxication with 3 µg/ml of cisplatin | 10 ng/ml | 6000.30 | 221.31 | 12 | 113 | p<0.001 |
| 7β-OH EPIA intoxication with 3 µg/ml of cisplatin | 100 nM | 4080.89 | 172.07 | 12 | 77 | p>0.05 |
| | 10 nM | 4275.04 | 125.14 | 12 | 80 | p>0.05 |
| | 1 nm | 4947.99 | 130.81 | 12 | 93 | p<0.001 |

### Protection by 7β-OH EPIA against cisplatin-induced neuronal cell death (loss of neuronal cell bodies)

### Incubation with 3µg/ml cisplatin for 48 hours

A mean of 61 sensory neurones per field was observed after incubation in medium together with "vehicle" without cisplatin at 48hours. Incubation with 3µg/ml cisplatin, reduced the number of neurones to a mean of 41 sensory neurones per field. This loss of neuronal cell bodies by cisplatin was statistically significant (-33 %, p<0.001) when compared to the number of cell bodies assessed after 48hours without cisplatin, i.e. medium containing vehicle only.

Incubation with 10 ng/ml NGF for 48hours almost completely prevented cisplatin-induced loss of cell bodies.

Incubation with 1nM, 10nM) and 100nM 7β-OH EPIA protected sensory neurones against cisplatin-induced cell death at 48hours. This effect was statistically significant. Total number of sensory neurones per field was 51, 45 and 50 after 48 hours of incubation with 1nM, 10nM) and 100nM 7β-OH EPIA respectively, which represents a reduction of cisplatin-induced cell death of 53,1%, 18,7% and 43,8% respectively.

### Incubation with 3µg/ml cisplatin for 72 hours

A mean of 54 sensory neurones per field was observed after incubation in medium together with "vehicle" without cisplatin at 72hours. Incubation with 3µg/ml cisplatin, reduced the number of neurones to a mean of 33 sensory neurones per field. This loss of neuronal cell bodies by cisplatin was statistically significant (-39 %, p<0.001) when compared to the number of cell bodies assessed after 72hours without cisplatin, i.e. medium containing vehicle only.

Incubation with 10ng/ml NGF for 48hours completely prevented cisplatin-induced loss of cell bodies.

Incubation with 1nM, and 10nM and 100nM 7β-OH EPIA almost completely protected sensory neurones against cisplatin-induced cell death at 72hours. This effect was statistically significant. Total number of sensory neurones per field was 52, 55 and 52 after 72 hours of incubation with 1nM, 10nM and 100nM 7β-OH EPIA, respectively, which represents a reduction of cisplatin-induced cell death of 93%, 104% and 93 % respectively.

**Table 11**

| Effects of vehicle (0.1% DMSO), NGF (10 ng/ml), and 7β-OH EPIA (1nM, 10nM and 100nM) on the number of cell bodies per field after 48 hours of incubation with cisplatin (3 µg/ml). | | | | | | |
|---|---|---|---|---|---|---|
| Treatment | Conc. | Average | SEM | n | % Control without cisplatin | p (compared to vehicle + cisplatin) |
| Vehicle (0.1% DMSO) | - | 60.83 | 1.59 | 12 | 100 | p<0.001 |
| Vehicle (0.1% DMSO) intoxication with 3 µg/ml of cisplatin | - | 40.75 | 1.88 | 12 | 67 | - |
| NGF intoxication with 3 µg/ml of cisplatin | 10 ng/ml | 58.33 | 1.38 | 12 | 96 | p<0.001 |
| 7β-OH EPIA intoxication with 3 µg/ml of cisplatin | 100 nM | 49.58 | 1.12 | 12 | 82 | p<0.001 |
| | 10 nM | 45.50 | 1.26 | 12 | 75 | p<0.05 |
| | 1 nM | 51.42 | 2.11 | 12 | 85 | p<0.001 |

**Table 12**

| Effect of the vehicle (0.1% DMSO), NGF (10 ng/ml) and 7β-OH EPIA (1 nM, 10 nM and 100nM) on the number of cell bodies per field after 72 hours of incubation with cisplatin (3 µg/ml). | | | | | | |
|---|---|---|---|---|---|---|
| Treatment | Conc. | Average | SEM | n | % Control without cisplatin | p (compared to vehicle + cisplatin) |
| Vehicle (0.1% DMSO) | - | 53.75 | 1.29 | 12 | 100 | p<0.001 |
| Vehicle (0.1% DMSO) intoxication with 3 µg/ml of cisplatin | - | 32.83 | 1.47 | 12 | 61 | - |
| NGF intoxication with 3 µg/ml of cisplatin | 10 ng/ml | 54.92 | 1.41 | 12 | 1.2 | p<0.001 |
| 7µ-OH EPIA intoxication with 3 µg/ml of cisplatin | 100 nM | 52.25 | 1.24 | 12 | 97 | p<0.001 |
| | 10 nM | 54.58 | 1.37 | 12 | 1.2 | p<0.001 |
| | 1 nM | 52.42 | 1.65 | 12 | 98 | p<0.001 |

### EXAMPLE 7

The present study evaluates the neurite growth-promoting effects of 7β-OH EPIA on dissociated spinal cord motor neurones and sensory neurones in primary cultures. Neurones were stained with anti β-Tubulin antibody and the analysis of the total neuronal length carried out using an "In Cell Analyzer". Neuronal cell cultures were incubated with 10⁻⁹M- 10⁻⁴M 7β-OH EPIA, and the neuronal network density was assessed, after 6 hours, 12 hours and 24 hours of incubation. Brain derived nerve growth factor (BDNF), a specific growth factor for motor neurones and nerve growth factor (NGF), a specific growth factor for sensory neurones, were used as reference compounds. Our data show that 7β-OH EPIA has marked neurotrophic effects, both on motor neurones and sensory neurones. These effects, which were comparable to the effects of BDNF and NGF, were particularly pronounced at nanomolar concentrations of the hydroxysteroid. Altogether, our findings support the uses of 7-hydroxysteroids for promoting neurite outgrowth and to treat peripheral neuropathy.

### 1. Materials and Methods

### 1.1. Preparation of rat Spinal cord motor neurone cell cultures

Rat spinal motor neurones were prepared according to the method described by Martinou et al., 1992 (Martinou JC, Martinou I, Kato AC Cholinergic differentiation factor (CDF/LIF) promotes survival of isolated rat embryonic motoneurons in vitro. Neuron. 1992 Apr;8(4):737-44). Briefly, pregnant female Wistar rats of 15 days gestation, were killed by cervical dislocation and the foetuses were removed from the uterus. Their spinal cords were removed and placed in ice-cold medium of Leibovitz (L15, Invitrogen, 11415-049) containing 1 % of bovine serum albuminutes (BSA fatty acid free, Eurobio, Les Ulis, France, GXXBSA01-65). Meninges were carefully removed.

The spinal motor neurones were dissociated by trypsinisation for 20 minutes at 37 °C (trypsin EDTA 10X Invitrogen 15400054) diluted in PBS without calcium and magnesium (Invitrogen 2007-03). The reaction was stopped by the addition of Dulbecco Modified Eagle's Media (DMEM, Invitrogen 21969-035) containing DNase I grade II (0.1 mg/ml Roche diagnostic 104159) and 10% foetal calf serum (FCS, Invitrogen 10270098). The suspension was then mechanically dissociated by 3 passages through a 10 ml pipette. Cells were then centrifuged at 580 g for 10 minutes at room temperature. The pellet of dissociated cells was re-suspended in L15 medium and the resulting suspension was enriched in motor neurones by centrifugation at 180 x g for 10 minutes at room temperature on a layer of BSA solution (3.5%) in L15 medium.

The supernatant was discarded and the pellet was re-suspended in L15 medium supplemented with DNase I (1%). Then the suspension was layered over a cushion of Optiprep (d: 1.06 g/ml; Abcys, Paris, France; 1030061) and centrifuged at 335 x g for 15 minutes at room temperature. The upper phase, containing the purified motor neurones, was collected, re-suspended with L15 medium and centrifuged at 800 x g for 10 minutes at room temperature. The cell pellet was finally resuspended in a defined culture medium consisting of neurobasal medium supplemented with 2% B27, 2 mM L-Glutamine and Penicillin 50 UI/ml - Streptomycin 50 µg/ml. Viable cells were counted in a Neubauer cytometer using the trypan blue exclusion test (Sigma T8154) then plated at 30 000 cells/well in 96 well-plates (pre-coated with poly-1-lysine; Nunclon, Invitrogen P5899) and cultured at 37 °C in a humidified air (95%) CO₂ (5%) atmosphere.

### 1.2. Preparation of rat Sensory neurone cell cultures

Rat sensory neurones were prepared according to the method described by Hall *et al.* 1997 (Hall AK, Ai X, Hickman GE, MacPhedran SE, Nduaguba CO, Robertson CP. The generation of neuronal heterogeneity in a rat sensory ganglion. J Neurosci. 1997 Apr 15;17(8):2775-84). Briefly, female rats (15 days gestation) were killed by cervical dislocation (Rats Wistar; Janvier, Le Genest-St-Isle, France) and the foetuses removed from the uterus. Their spinal cords with the dorsal root ganglia (DRG) were removed and placed in ice-cold medium of Leibovitz (L15, Fisher 11415-049) containing Penicillin 50 UI/ml - Streptomycin 50 µg/ml (PS, 1%) and bovine serum albuminutes (BSA 1 %, Sigma A6003). The DRG were recovered and dissociated by trypsinisation for 20 minutes at 37 °C (trypsin EDTA 10X, 10 %, Fisher 15400054) diluted in PBS without calcium and magnesium (Fisher 2007-03). The reaction was stopped by addition of Dulbecco modified Eagle medium (DMEM, Fisher 21969-035) containing DNase I grade II (0.1 mg/ml Roche diagnostic 104159) and foetal bovine serum (FBS 10%, Fisher 10270-098). The cell suspension was triturated with a 10 ml pipette and centrifuged at 350 x g for 10 minutes at room temperature. The pellet of dissociated cells was then resuspended in defined culture medium.

Viable cells were counted in a Neubauer cytometer using the trypan blue exclusion test (Sigma) and seeded at a density of 25 000 cells/well in 96 well-plates (Nunc). Wells were pre-coated with poly-L-lysine (10 µg/ml, Sigma P2636) in ultra pure sterile water (Merck Eurolab 60759.01).

Cells were allowed to adhere for 2 hours and maintained in a humidified incubator at 37 °C in 5% CO₂/95% air atmosphere.

### 1.3. Incubation of Spinal cord motor neurone cell cultures with active compounds

The culture medium was changed into a defined culture medium after a 12 hours incubation period following the conditions described below:

Control (vehicle, 0.1% DMSO 0.1%)

7β-OH EPIA 10⁻⁴ M, 10⁻⁵ M, 10⁻⁶ M, 10⁻⁷M, 10⁻⁸M and 10⁻⁹M (in 0.1% DMSO)

BDNF 50 ng/ml and 10 ng/ml (in 0.1% DMSO)

After 6 hours, 12 hours and 24 hours of incubation with, cells were fixed for 5 minutes in an ethanol/acetic acid solution (95%/5%) at -20°C and rinsed 3 times in PBS.

### 1.4. Incubation of Sensory neurone cell cultures with active compounds

The culture medium was changed into a defined culture medium after a 12 hours incubation period following the conditions described below:

Control (vehicle, 0.1% DMSO)

7β-OH EPIA - 10⁻⁴ M, 10⁻⁵ M, 10⁻⁶M, 10⁻⁷M, 10⁻⁸M and 10⁻⁹M (in 0.1% DMSO)

NGF 50 ng/ml and 10 ng/ml (in 0.1% DMSO)

After 6 hours, 12 hours and 24 hours of incubation with 7β-OH EPIA, cells were fixed for 5 minutes in an ethanol/acetic acid solution (95%/5%) at -20°C and rinsed 3 times in PBS.

### 1.5. Analysis of neurite growth

Motor neurones and sensory neurones were labelled by monoclonal anti β -Tubulin antibody (Sigma T8660) diluted at 1:400 in incubation solution (PBS with 5 % of FCS and 0.1 % of saponin, Sigma S-7900). This antibody specifically labels neurone cell bodies and neurites.

After 2 hours of incubation, the cells were washed in PBS and incubated with Alexa Fluor 488 goat anti mouse IgG (Molecular Probes A11001) diluted at 1:300 in incubation solution to reveal the β-Tubulin III antibodies.

Analysis of the total length of neurones labelled with anti β-Tubulin antibodies (neurones) were carried out using In Cell Analyzer 1000 3.2.Workstation software.

The results were expressed in percentage compared to the vehicle. Comparison of groups was were carried out using the unpaired T test.

### 2- RESULTS

### 2.1. Effect of drug treatment on neurite length of motor neurones

Analysis of the total length of cell extensions gives an indication of the neurotrophic properties of the test compounds.
a) After 6 hours of incubation

The results are shown in Table 13. Treatment with BDNF at 50 ng/ml and 10 ng/ml significantly increased the density of the neurite network formed by motor neurones by respectively 180 % and 193 % compared to the vehicle (p<0.001).

Incubation with 10⁻⁸ M and 10⁻⁹ M for 7β-OH EPIA 6 hours significantly increased the neurite network density of motor neurones, respectively 150 % and 188 % when compared to the vehicle (p<0.001).
b) After 12 hours of incubation

The results are shown in Table 14. Treatment with 50 ng/ml and 10 ng/ml BDNF significantly increased the density of the neurite network formed by motor neurones, respectively 152 % and 173 % when compared to the vehicle (p<0.001).

Incubation with 10⁻⁴ M to 10⁻⁹ M 7β-OH EPIA for 12 hours significantly increased the neurite network density, 226 % to 137% when compared to the vehicle.
c) After 24 hours of incubation

The results are shown in Table 15. 24 hours of incubation with 10 ng/ml BDNF significantly increased the neurite network density when compared to vehicle (170 %, p<0.01). However, incubation with 50 ng/ml BDNF did not significantly modify the neurite network density.

Incubation with 10⁻⁸ M 7β-OH EPIA for 24 hours increased neurite network density by 174 % when compared to vehicle (p<0.005).

### 2.2. Effect of drug treatment on neurite length of sensory neurones

a) After 6 hours of incubation

The results are shown in Table 16. Treatment with 50 ng/ml and 10 ng/ml NGF for 6 hours significantly increased the density of the neurite network formed by sensory neurones by respectively 273 % (p<0.001) and 191 % (p<0.01).

Incubation with 10⁻⁷ M, 10⁻⁸ M and 10⁻⁹ M 7β-OH EPIA for 6 hours increased the neurite network density of sensory neurones by respectively 171 % (p<0.001), 176 %, (p<0.005) and 149% (p<0.05) when compared to control.
b) After 12 hours of incubation.

The results are shown in Table 17. Incubation with 50 ng/ml and 10 ng/ml NGF significantly increased the density of the neurite network formed by sensory neurones compared to the vehicle by respectively 216 % (p<0.001) and 128 % (p<0.05).

Incubation with 10⁻⁹ M and 10⁻⁷ M 7β-OH EPIA significantly increased the neurite network density when compared to the vehicle, by respectively 145 % (p<0.001) and 134 % (p<0.05).
c) After 24 hours of incubation

The results are shown in Table 18. After 24 hours of Incubation, with 50 ng/ml and 10 ng/ml NGF significantly increased the density of the neurite network formed by sensory neurones, by respectively 309 % and 371 % when compared to the vehicle (p<0.001).

After 24 hours of Incubation with 10⁻⁷ M, 10⁻⁸ M and 10⁻⁹ M 7β-OH EPIA for 24hours increased the neurite network density compared when to the vehicle by respectively 173 % (p<0.005), 174 % (p<0.01) and 147 % (p<0.05).

**Table 13**

| **Motor neurones - Neurites length per field after 6 hours of incubation** | | | | | | |
|---|---|---|---|---|---|---|
| Treatment | Conc. | Mean (µm) | sem | n | % Vehicle | *P* |
| Vehicle (0.1% DMSO) | - | 254.3 | 19.0 | 12 | 100 | - |
| BDNF | 50 ng/ml | 457.2 | 19.4 | 12 | 180 | *p<0.001* |
| | 10 ng/ml | 489.5 | 19.0 | 12 | 193 | *p<0.001* |
| 7β-OH-EPIA | 10⁻⁴ M | 315.9 | 18.9 | 12 | 124 | *p<0.05* |
| | 10⁻⁵ M | 293.3 | 22.8 | 12 | 115 | *p<0.05* |
| | 10⁻⁶ M | 354.5 | 33.7 | 12 | 139 | *p<0.05* |
| | 10⁻⁷ M | 350.5 | 34.3 | 12 | 138 | *p<0.05* |
| | 10⁻⁸ M | 382.4 | 25.8 | 12 | 150 | *p*<0.001 |
| | 10⁻⁹ M | 479.3 | 40.8 | 12 | 188 | *p*<0.001 |

**Table 14**

| **Motor neurones - Neurites length per field after 12 hours of incubation** | | | | | | |
|---|---|---|---|---|---|---|
| Treatment | Conc. | Mean (µm) | sem | n | % Vehicle | *P* |
| Vehicle (0.1% DMSO) | - | 423.8 | 38.3 | 12 | 100 | - |
| BDNF | 50 ng/ml | 643.7 | 34.1 | 12 | 152 | *p<0.001* |
| | 10 ng/ml | 731.3 | 34.8 | 12 | 173 | *p*<*0.001* |
| 7β-OH-EPIA | 10⁻⁴ M | 602.4 | 24.8 | 12 | 142 | *p*<*0.001* |
| | 10⁻⁵ M | 680.9 | 30.8 | 12 | 161 | *p*<*0.001* |
| | 10⁻⁶ M | 580.3 | 55.5 | 12 | 137 | *p*<*0.05* |
| | 10⁻⁷ M | 956.7 | 54.6 | 12 | 137 | *p*<*0.001* |
| | 10⁻⁸ M | 759.0 | 61.6 | 12 | 179 | *p*<*0.001* |
| | 10⁻⁹ M | 764.9 | 59.9 | 12 | 180 | *p*<*0.001* |

**Table 15**

| **Motor neurones - Neurites length per field after 24 hours of incubation** | | | | | | |
|---|---|---|---|---|---|---|
| Treatment | Conc. | Mean (µm) | sem | n | % Vehicle | *P* |
| Vehicle (0.1 % DMSO) | - | 215.8 | 39.6 | 12 | 100 | - |
| BDNF | 50 ng/ml | 293.6 | 20.1 | 12 | 136 | *p*<*0.001* |
| | 10 ng/ml | 366.1 | 29.6 | 12 | 170 | *p*<*0.001* |
| 7β-OH-EPIA | 10⁻⁴ M | 188.4 | 23.3 | 12 | 87 | *p*<*0.05* |
| | 10⁻⁵ M | 200.9 | 20.7 | 12 | 93 | *p*<*0.05* |
| | 10⁻⁶ M | 224.8 | 36.7 | 12 | 104 | *p*<*0.05* |
| | 10⁻⁷ M | 271.7 | 34.0 | 12 | 126 | *p*<*0.05* |
| | 10⁻⁸ M | 374.6 | 22.3 | 12 | 174 | *p*<*0.005* |
| | 10⁻⁹ M | 265.9 | 26.2 | 12 | 123 | *p*<*0.05* |

**Table 16**

| **Sensory neurones - Neurites length per field after 6 hours of incubation** | | | | | | |
|---|---|---|---|---|---|---|
| Treatment | Conc. | Mean (µM) | sem | n | % Vehicle | *P* |
| Vehicle (0.1% DMSO) | - | 102.2 | 14.1 | 12 | 100 | - |
| NGF | 50 ng/ml | 279.1 | 31.8 | 12 | 273 | *p*<*0.001* |
| | 10 ng/ml | 195.2 | 28.8 | 12 | 191 | *p*<*0.01* |
| 7β-OH-EPIA | 10⁻⁴ M | 103.2 | 10.7 | 12 | 101 | *p<0.05* |
| | 10⁻⁵ M | 114.8 | 8.2 | 12 | 112 | *p<0.05* |
| | 10⁻⁶ M | 97.7 | 11.4 | 12 | 96 | *p<0.05* |
| | 10⁻⁷ M | 151.8 | 11.7 | 12 | 149 | *p<0.05* |
| | 10⁻⁸ M | 179.3 | 16.0 | 12 | 176 | *p<0.005* |
| | 10⁻⁹ M | 174.2 | 9.1 | 12 | 171 | *p*<*0.001* |

**Table 17**

| **Sensory neurones - Neurites length per field after 12 hours of incubation** | | | | | | |
|---|---|---|---|---|---|---|
| Treatment | Conc. | Mean (µm) | sem | n | % Vehicle | *P* |
| Vehicle (0.1% DMSO) | - | 298.4 | 12.8 | 12 | 100 | - |
| NGF | 50 ng/ml | 644.3 | 40.0 | 12 | 216 | *p*<*0.001* |
| | 10 ng/ml | 381.3 | 31.4 | 12 | 128 | *p<0.05* |
| 7β-OH-EPIA | 10⁻⁴ M | 290.3 | 32.3 | 12 | 97 | *p<0.05* |
| | 10⁻⁵ M | 321.0 | 18.4 | 12 | 108 | *p<0.05* |
| | 10⁻⁶ M | 287.0 | 24.3 | 12 | 96 | *p<0.05* |
| | 10⁻⁷ M | 398.4 | 35.8 | 12 | 134 | *p<0.05* |
| | 10⁻⁸ M | 368.3 | 32.2 | 12 | 123 | *p<0.05* |
| | ¹⁰⁻⁹ M | 431.8 | 24.2 | 12 | 145 | *p*<*0.001* |

**Table 18**

| **Sensory neurones - Neurites length per field after 24 hours of incubation** | | | | | | |
|---|---|---|---|---|---|---|
| Treatment | Conc. | Mean (µm) | sem | n | % Vehicle | *P* |
| Vehicle (0.1% DMSO) | - | 220.7 | 12.4 | 12 | 100 | - |
| NGF | 50 ng/ml | 682.1 | 64.2 | 12 | 309 | *p<0.001* |
| | 10 ng/ml | 819.9 | 61.5 | 12 | 371 | *p<0.001* |
| 7β-OH-EPIA | 10⁻⁴ M | 228.6 | 38.8 | 12 | 104 | *p<0.05* |
| | 10⁻⁵ M | 244.8 | 28.2 | 12 | 111 | *p<0.05* |
| | 10⁻⁶ M | 189.9 | 24.4 | 12 | 86 | *p<0.05* |
| | 10⁻⁷ M | 380.8 | 45.8 | 12 | 173 | *p<0.005* |
| | 10⁻⁸ M | 384.3 | 52.2 | 12 | 174 | *p<0.01* |
| | 10⁻⁹ M | 324.9 | 45.1 | 12 | 147 | *p<0.05* |

## Claims

1. The use of a compound of formula (IV): 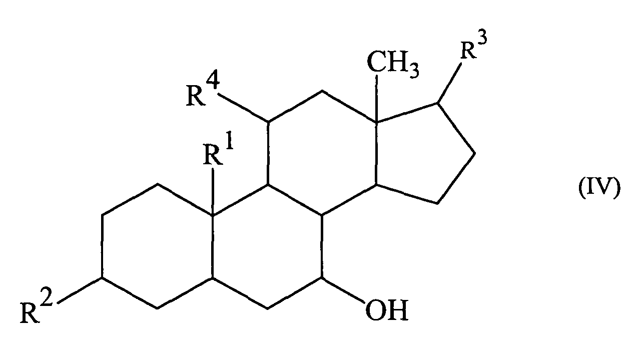 wherein:
R¹ represents a hydrogen atom or a methyl group; and
R², R³ and R⁴ are the same as or different from each other and each represents an oxo group, a hydroxy group, a mercapto group, a hydrogen atom, a halogen atom, an alkoxy group, an aryloxy group or an acyl group;
or a pharmaceutically acceptable salt or ester thereof,
as an agent which enhances production of 15-deoxy-prostaglandin J2 for the manufacture of a medicament for the treatment or prophylaxis of conditions mediated by enhanced levels of prostaglandin E2 or other metabolites of cyclooxygenase and prostaglandin synthase activity or for the treatment or prophylaxis of conditions made worse due to a reduced level or reduced availability of 15-deoxy-prostaglandin J2.

2. The use of a compound of formula (IV) as defined in Claim 1 as an agent which facilitates production of 15-deoxy-prostaglandin J2 and selectively inhibits production of prostaglandin E2 in the presence of an inflammation causing agent for the manufacture of a medicament for the treatment or prophylaxis of conditions mediated by enhanced levels of prostaglandin E2 or other metabolites of cyclooxygenase activity or for the treatment or prophylaxis of conditions made worse due to a reduced level or reduced availability of 15-deoxy-prostaglandin J2.

3. The use of a compound of formula (IV) as defined in Claim 1 as an agent which enhances the production of 15-deoxy-prostaglandin J2 for the manufacture of a medicament to promote neurite outgrowth or to treat peripheral neuropathy.

4. The use of a compound of formula (IV) as defined in Claim 1 as an agent which enhances the production of 15-deoxy-prostaglandin J2 and in turn activates PPARgamma for the manufacture of a medicament to treat a condition requiring the activation of PPARgamma.

5. The use of a compound of formula (IV) as defined in Claim 1 for the manufacture of a medicament for the treatment or prophylaxis of: diabetes mellitus and its sequelae; ischemic vascular diseases; pain associated with inflammation; inflammatory skin conditions; spinal cord injury; peripheral neuropathy; multiple sclerosis; inflammatory bowel disease; rheumatoid arthritis, metabolic syndrome X, obesity, acromegaly and wound healing.

6. The use of a compound of formula (IV) as defined in Claim 1 as an agent which enhances the production of 15-deoxy-prostaglandin J2 for the manufacture of a medicament for the treatment or prophylaxis of cancer.

7. The use of a compound of formula (IV) as defined in Claim 1 as an agent which enhances the production of 15-deoxy-prostaglandin J2 for the manufacture of a medicament for inhibiting cancer cell proliferation, inducing apoptosis in cancer cells, or inhibiting tumour growth and progression.

8. The use according to any one of Claims 6 and 7, in which the cancer is colorectal, gastric, breast, hepatic, prostate, bladder, thyroid papillary or oesophageal cancer.

9. The use of an agent according to Claim 1 or 2 wherein the condition includes inflammation or inflammatory diseases of peripheral organs.

10. The use according to Claim 9 wherein the peripheral organs include liver or kidney.

11. The use according to Claim 1 or 2 wherein the condition includes inflammatory airway diseases.

12. The use according to Claim 11 wherein the inflammatory airway disease includes asthma, rhinitis, bronchitis, or chronic obstructive pulmonary disease.

13. The use according to any one of Claims 1 to 4 for the treatment and prophylaxis of:
pain associated with inflammation;
peripheral arterial diseases and their sequelae such as critical limb ischaemia;
coronary artery disease and its sequelae;
cerebrovascular diseases and its sequelae;
liver and kidney ischaemia;
metabolic diseases;
obesity and its sequela;
inflammatory airways diseases;
chronic neurodegenerative diseases;
acute neurological degenerative conditions;
inflammatory bowel disease;
inflammatory diseases **characterised by** degeneration of articular cartilage; and
wound healing.

14. The use according to any one of Claims 1 to 4 for the treatment and prophylaxis of:
pain associated with inflammation;
critical limb ischaemia;
ischaemic heart disease and myocardial infarction;
stroke and transient ischaemic attacks;
atherosclerotic renal artery stenosis;
type 2 diabetes and its sequelae, peripheral arterial diseases, coronary artery disease, vascular diseases of the kidney and diabetic neuropathy;
asthma and chronic obstructive pulmonary disease;
Alzheimer's disease, Parkinson's disease, multiple sclerosis and peripheral neuropathies;
traumatic brain injury and spinal cord injury;
inflammatory bowel disease; and
rheumatoid arthritis and primary and secondary osteoarthritis and their sequelae.
15. The use according to Claim 3 or Claim 5, wherein the peripheral neuropathy is caused by a chemotherapeutic agent.
16. The use according to Claim 15, wherein the chemotherapeutic agent is cisplatin.
17. The use according to any one of Claims 1 to 16, in which said compound of formula (IV) is 7α- or 7β-hydroxyepiandrosterone or an ester thereof.
